# EUROPÄISCHE PATENTSCHRIFT

(11) **EP 2 352 835 B1**
(45) Veröffentlichungstag und Bekanntmachung des Hinweises auf die Patenterteilung: **05.09.2012**
(21) Anmeldenummer: 09776142.3
(22) Anmeldetag: 07.07.2009
(51) Int. Cl.: C12P 5/02, C02F 11/04, C12N 1/20

(54) **Clostridium sporosphaeroides zur Erzeugung von Biogas aus Biomasse**
Clostridium sporosphaeroides for production of biogas from biomass
Clostridium sporosphaeroides pour la production de biogaz à partir de la biomasse

(30) Priorität: 23.12.2008 WO PCT/DE2008/075017; 07.03.2009 DE 102009003587
(43) Veröffentlichungstag der Anmeldung: 10.08.2011
(73) Patentinhaber: Schmack Biogas GmbH, 92421 Schwandorf (DE)
(72) Erfinder: REUTER, Monika, 84561 Mehring (DE); VATER, Daniel, 02953 Bad Muskau (DE); DUCHOW, Vera, 88471 Laupheim (DE)
(74) Vertreter: Graf Glück Habersack Kritzenberger
(86) Internationale Anmeldenummer: PCT/DE2009/075035
(87) Internationale Veröffentlichungsnummer: WO 2010/072219

(56) Entgegenhaltungen:
- WO-A-2006/056819
- WO-A-2006/119052
- WO-A-2007/052306
- CA-A1- 2 258 254
- NIELSEN HENRIK BANGSO ET AL: "Bioaugmentation of a two-stage thermophilic (68 degrees C/55 degrees C) anaerobic digestion concept for improvement of the methane yield from cattle manure" BIOTECHNOLOGY AND BIOENGINEERING, Bd. 97, Nr. 6, August 2007 (2007-08), Seiten 1638-1643, XP002538156 ISSN: 0006-3592
- DATABASE EMBL [Online] 21. März 2007 (2007-03-21), "Uncultured Clostridiales bacterium clone 1099982248072 16S ribosomal RNA gene, partial sequence" XP002538673 Database accession no. EF434352
- LIU W-T ET AL: "Characterization of microbial community in granular sludge treating brewery wastewater" WATER RESEARCH, ELSEVIER, AMSTERDAM, NL, Bd. 36, Nr. 7, 1. April 2002 (2002-04-01), Seiten 1767-1775, XP004347949 ISSN: 0043-1354
- KRÖBER MAGDALENA ET AL: "Phylogenetic characterization of a biogas plant microbial community integrating clone library 16S-rDNA sequences and metagenome sequence data obtained by 454-pyrosequencing." JOURNAL OF BIOTECHNOLOGY, Bd. 142, Nr. 1, 1. Juni 2009 (2009-06-01), Seiten 38-49, XP002538671 ISSN: 1873-4863
- SNEATH P H A ET AL (EDS.): "Bergey's manual of systematic bacteriology, Volume 2, Section 13: Endospore-forming Gram-positive rods and cocci" 1986, WILLIAMS & WILKINS, BALTIMORE, USA , XP002538672 Seite 1151; Tabelle 13.13 Seiten 1192-93, Absatz 70.
- DATABASE BIOSIS [Online] BIOSCIENCES INFORMATION SERVICE, PHILADELPHIA, PA, US; 1985, QUENTMEIER A ET AL: "CHARACTERIZATION OF CITRATE LYASE FROM CLOSTRIDIUM-SPOROSPHAEROIDES" XP002538674 Database accession no. PREV198579108063 & ARCHIVES OF MICROBIOLOGY, Bd. 141, Nr. 1, 1985, Seiten 85-90, ISSN: 0302-8933
- WILDE E ET AL: "Clostridium pascui sp. nov., a new glutamate-fermenting sporeformer from a pasture in Pakistan." INTERNATIONAL JOURNAL OF SYSTEMATIC BACTERIOLOGY, Bd. 47, Nr. 1, Januar 1997 (1997-01), Seiten 164-170, XP002538670 ISSN: 0020-7713
- STENGL S ET AL: "Betriebsstabilität von Biogasanlagen" ETA ENERGIE, [Online] Nr. 3, 2007, Seiten 30-33, XP002562310 Succidia AG, Darmstadt, Deutschland Gefunden im Internet: URL:http://www.succidia.de/n_energie.html> [gefunden am 2007-01-07]
- SNEATH P H A ET AL: "Bergey's manual of systematic bacteriology, Endospore-forming Gram-positive rods and cocci" BERGEY'S MANUAL OF SYSTEMATIC BACTERIOLOGY, WILLIAMS & WILKINS, BALTIMORE, USA, Bd. 2, 1. Januar 1986 (1986-01-01), Seiten 1154,1187-1188, XP002538393 ISBN: 978-0-683-07893-0
- HU B ET AL: "Changes in microbial community composition following treatment of methanogenic granules with chloroform" ENVIRONMENTAL PROGRESS & SUSTAINABLE ENERGY, JOHN WILEY & SONS, INC, US, Bd. 28, Nr. 1, 1. April 2009 (2009-04-01), Seiten 60-71, XP002538843 ISSN: 1944-7442 [gefunden am 2009-02-17]
- SNEATH P H A ET AL: "Bergey's manual of systematic bacteriology, Endospore-forming Gram-positive rods and cocci" BERGEY'S MANUAL OF SYSTEMATIC BACTERIOLOGY, WILLIAMS & WILKINS, BALTIMORE, USA, Bd. 2, 1. Januar 1986 (1986-01-01), Seiten 1118,1123-1124,1133, XP002539295 ISBN: 978-0-683-07893-0
- VAN HEERDEN I ET AL: "Microbial, chemical and physical aspects of citrus waste composting" BIORESOURCE TECHNOLOGY, ELSEVIER BV, GB, Bd. 81, Nr. 1, 1. Januar 2002 (2002-01-01), Seiten 71-76, XP002539293 ISSN: 0960-8524
- RYCKEBOER J ET AL: "Microbiological aspects of biowaste during composting in a monitored compost bin" JOURNAL OF APPLIED MICROBIOLOGY, OXFORD, GB, Bd. 94, Nr. 1, 1. Januar 2003 (2003-01-01), Seiten 127-137, XP002539294 ISSN: 1364-5072
- "C. sartagoforme-Einsatz in Biogasanlagen", ISF SCHAUMANN FORSCHUNG, 13 January 2012 (2012-01-13), pages 1-6, XP007920122,

## Beschreibung

### Technisches Gebiet

Die Erfindung betrifft ein Verfahren zur Erzeugung von Biogas aus Biomasse unter Verwendung eines Mikroorganismus der Art *Clostridium sporosphaeroides.*

### Stand der Technik

Biokraftstoffe werden hauptsächlich durch Vergärung von Pflanzensubstraten mit Hilfe von Hefen, Bakterien oder Pilzen gewonnen. Die produzierten flüssigen Energieträger, insbesondere Bioethanol, können anschließend als Brennstoff in geeigneten Feuerungsanlagen oder als Treibstoff oder -zusatz in Kraftfahrzeugmotoren oder Motoren zur Strom- und Wärmeerzeugung eingesetzt werden. Die Biomasse wird somit in einen flüssigen und gut transportierbaren Energieträger mit relativ hoher Energiedichte umgewandelt, der dann weitgehend universell eingesetzt werden kann.

Als Rohstoffe für die Erzeugung von flüssigen Biokraftstoffen kommen meist lokal verfügbare Pflanzen mit einem hohen Gehalt an Zucker oder Stärke zum Einsatz. In Europa sind dies vor allem Mais- und Getreidekörner sowie Zuckerrüben, in Nordamerika Mais und in Lateinamerika Zuckerrohr oder -melasse. In Frage kommen aber auch weitere Energiepflanzen wie Zuckerhirse (Sorghum), Triticale oder Cassava (Maniok). Derzeit werden als Ausgangsmaterial bei der Verflüssigung von Biomasse insbesondere Maiskorn und Getreidekorn eingesetzt. Aufgrund der zunehmenden Rohstoffknappheit, der gestiegenen Rohstoffpreise, einer enstandenen Konkurrenzsituation um Pflanzen, die sowohl der menschlichen und tierischen Nahrung dienen als auch der Energiegewinnung, einer verbesserten CO₂-Bilanz sowie der Schonung der Ressourcen wird nun zunehmend versucht, nicht nur die Körner von Mais und Getreide zu verwenden, sondern vielmehr die gesamte Pflanze der Energiegewinnung zuzuführen oder Pflanzen zu verwenden, die keiner intensiven landwirtschaftlichen Bewirtschaftung bedürfen wie Rutenhirse oder Chinaschilf. Bei den aus diesen Rohstoffen hergestellten Biokraftstoffen handelt es sich um sogenannte "second generation"-Kraftstoffe. Daneben wird auch eine vermehrte Nutzung von günstigen pflanzlichen Reststoffen wie Stroh, pflanzlichen Abfällen aus der Holzwirtschaft oder Garten- und Landschaftspflege angestrebt.

Um eine effiziente Vergärung der Biomasse sicher zu stellen, muss das als Rohstoff eingesetzte pflanzliche Material und insbesondere die darin enthaltene organische Trockensubstanz (oTS) für eine anschließende Verwertung aufgeschlossen werden. Dies geschieht durch Hydrolyse der organischen Trockensubstanz, was gleichermaßen einer Verflüssigung der organischen Trockensubstanz entspricht. Aus dem flüssigen Biomasse-Substrat wird dann durch Vergärung Ethanol oder ein anderer Biokraftstoff hergestellt. Mit den vorhandenen Techniken wird aber keine hohe Energieeffizienz erreicht, was insbesondere an der nicht ausreichenden Verflüssigung und damit Verwertung des Rohmaterials liegt.

Bei der Herstellung von Bioethanol erfolgt die ethanolische Gärung in erster Linie durch zugesetzte Hefen, die Glucose in Ethanol umwandeln. Je nach Substrat ist schon ein relativ hoher Zuckeranteil vorhanden (z.B. bei Melasse) oder es müssen zuerst höhermolekulare Substrate wie Stärke, Cellulose oder Hemicellulose (z.B. bei Getreide, Stroh, Ganzpflanzeneinsatz) enzymatisch oder chemisch gespalten werden, damit die alkoholische Gärung erfolgen kann. Für diesen hydrolytischen Substrataufschluss, der auch einer Verflüssigung entspricht, sind in erster Linie Mikroorganismen, insbesondere Bakterien verantwortlich.

Soll die Umwandlung organischer Rohstoffe nicht zu einem flüssigen Biokraftstoff, sondern zu einem gasförmigen Energieträger führen, so kommen Biogasanlagen zum Einsatz. In Biogasanlagen wird Methan durch einen mikrobiellen Abbauprozess organischer Substanzen erzeugt. Das Biogas entsteht dabei in einem mehrstufigen Prozess der Vergärung oder Faulung durch die Aktivität von anaeroben oder mikroaerophilen Mikroorganismen, d.h. unter Ausschluss von Luft.

Das als Gärsubstrat verwendete organische Material besitzt aus chemischer Sicht einen hochmolekularen Aufbau, der in den einzelnen Verfahrensschritten einer Biogasanlage durch Stoffwechseltätigkeit der Mikroorganismen zu niedermolekularen Bausteinen abgebaut wird. Die bei der Fermentierung des organischen Gärsubstrats aktiven Populationen von Mikroorganismen sind bislang aber nur unzureichend charakterisiert.

Nach heutigem Kenntnisstand können vier nacheinander, aber auch parallel ablaufende und ineinander greifende biochemische Stoffwechselprozesse unterschieden werden, die den Abbau von organischen Gärsubstraten zu den Endprodukten Methan und Kohlendioxid ermöglichen: Hydrolyse, Acidogenese, Acetogenese und Methanogenese.

In der Hydrolyse werden hochmolekulare, oft partikulär vorliegende, organische Verbindungen durch Exoenzyme (z.B. Cellulasen, Amylasen, Proteasen, Lipasen) fermentativer Bakterien in lösliche Spaltprodukte überführt. Dabei werden beispielsweise Fette in Fettsäuren, Kohlenhydrate, wie z.B. Polysaccharide, in Oligo- und Monosaccharide sowie Proteine in Oligopeptide beziehungsweise Aminosäuren zerlegt. Die daneben gebildeten gasförmigen Produkte bestehen überwiegend aus Kohlendioxid.

Fakultativ und obligat anaerob lebende Bakterien, oftmals identisch mit den hydrolysierenden Bakterien, verstoffwechseln in der Acidogenese die Hydrolyseprodukte (z.B. Mono-, Disaccharide, Di-, Oligopeptide, Aminosäuren, Glycerin, langkettige Fettsäuren) intrazellulär zu kurzkettigen Fett- oder Carbonsäuren, wie beispielsweise Butter-, Propion- und Essigsäure, zu kurzkettigen Alkoholen wie zum Beispiel Ethanol und zu den gasförmigen Produkten Wasserstoff und Kohlendioxid.

In der sich anschließenden Acetogenese werden die in der Acidogenese gebildeten kurzkettigen Fett- und Carbonsäuren sowie die kurzkettigen Alkohole von acetogenen Bakterien aufgenommen und nach β-Oxidation als Essigsäure wieder ausgeschieden. Nebenprodukte der Acetogenese sind CO₂ und molekularer Wasserstoff (H₂).

Die Produkte der Acetogenese wie Essigsäure aber auch andere Substrate wie Methanol und Formiat werden durch Methan-bildende Organismen in der obligat anaerob verlaufenden Methanogenese zu Methan und CO₂ umgesetzt. Das hier entstehende CO₂ und auch das während der übrigen Prozessschritte, wie z.B. der Hydrolyse, gebildete CO₂ kann wiederum durch Mikroorganismen mit dem angefallenen H₂ ebenfalls zu Methan umgesetzt werden.

Die Erhöhung der Ausbeute an Endprodukten aus einer gegebenen Menge an Edukten stellt wie bei jeder chemischen Umsetzung auch sowohl im Fall der Verflüssigung von Biomasse wie auch bei der Herstellung von Biogas ein vordringliches Ziel der Prozessführung dar. Aus einer gegebenen Menge an Biomasse soll eine möglichst große Menge an flüssigen organischen Verbindungen bzw. eine möglichst große Menge an Methan gebildet werden.

Die Fachagentur Nachwachsende Rohstoffe hat im Jahr 2005 Ergebnisse eines Biogas-Messprogramms veröffentlicht ("Ergebnisse des Biogas-Messprogramms", 2005, Hrsgb. Fachagentur Nachwachsende Rohstoffe e.V., Gülzow), worin verschiedene Biogasanlagen verglichen wurden. Dieses Messprogramm stellt einen repräsentativen Querschnitt der bestehenden Anlagenvielfalt dar, da Biogasanlagen verschiedener Bauarten, verschiedener Hersteller und mit verschiedenen Einsatzstoffen betriebene Anlagen erfasst wurden. Die gemessenen Gesamtraumbelastungen der untersuchten Biogasanlagen lagen in einem Bereich zwischen 0,45 kgoTS/m³d (mehrstufige Anlagen) und 5,7 kgoTS/m³d (einstufige Anlagen). Sowohl für die meisten ein- als auch mehrstufigen Anlagen lag die Raumbelastung zwischen 1 und 3 kgoTS/m³d.

Unter der Raumbelastung eines Fermenters versteht man die Menge an dem Fermenter zugeführten Substrat, die in Kilogramm organischer Trockensubstanz pro Kubikmeter Fermentervolumen und pro Tag angegeben wird. Die Menge an erzeugtem Biogas hängt stark von der Raumbelastung des Fermenters ab, wobei mit steigender Raumbelastung eine zunehmend größere Menge an Biogas erzeugt wird. Eine hohe Raumbelastung macht den Prozess der Biogaserzeugung also zunehmend ökonomisch rentabler, führt andererseits aber zu einer zunehmenden Destabilisierung der biologischen Prozesse der Fermentierung.

Die Steigerung der Raumbelastung ist eine Möglichkeit, um eine Biogasanlage effizienter zu betreiben. Dabei wird durch eine langsame Steigerung der Füttermenge in oTS pro Tag die Biogasproduktion erhöht. In der Praxis kommen hohe Raumbelastungen von über 6 kgoTS/m³d bislang nicht vor. Ziel im Anlagenbetrieb ist es, eine Anlage wirtschaftlich zu betreiben. Dabei steht die Stabilität des biologischen Prozesses an vorderster Stelle, da ein Anlagenausfall extrem hohe Kosten verursacht.

Um diese Prozessstabilität zu erreichen, werden die bestehenden Anlagen bei niedrigen Raumbelastungen betrieben, was aber ein entsprechend großes Gärvolumen für einen effizienten Betrieb notwendig macht. Dadurch werden die Anfangsinvestitionen durch zusätzliche Baukosten erhöht. Bei hohen Raumbelastungen kommt es aufgrund einer stärkeren Belastung der im Fermenter existierenden Biozönose mit organischem Material vor allem in Form von Feststoffen zu einer kontinuierlichen Eindickung des Fermenterinhalts. Bedingt wird dies durch steigende Trockensubstanzgehalte und einer unvollständigen Umwandlung der Biomasse in Biogas.

Substrate für die Biogasherstellung weisen Trockensubstanzanteile im Bereich von 5 % bis 90 % auf. Für Nassgärverfahren, bei denen pumpfähige Substrate benötigt werden, können hierbei Substrate mit einem Trockensubstanzanteil bis etwa 35 bis höchstens 40 % eingesetzt werden. Da Substrate mit einem höhreren Trockensubstanzanteil, insbesondere einem höheren Anteil an organischer Trockensubstanz, meist einen höheren Energiegehalt liefern, würden diese bevorzugt eingesetzt. Entgegen stehen in diesem Fall jedoch höhere Kosten durch vermehrten Energieaufwand beim Pumpen und Rühren sowie höhere Nebenkosten durch Abnutzung oder Reparatur von Pumpen, Rührwerken o.ä. bei hoher Viskosität oder einem hohen Trockensubstanzgehalt. Falls eine Substratverdünnung wegen eines hohen Trockensubstanzgehalts notwendig ist, entstehen zusätzliche Wasserund Abwasserkosten sowie technische Ausrüstung zur Wasserzufuhr, - rückgewinnung oder Abwasserbehandlung.

Ein im kontinuierlichen Betrieb steigender Trockensubstanzanteil führt zunehmend zu Problemen bei der Rühr- und Pumpfähigkeit des Fermenterinhalts. Bei einer weiteren Steigerung der Trockensubstanzgehalte kann diese Eindickung zur Instabilität der Biozönose und damit zum weitgehenden bis vollständigen Erliegen der biologischen Prozesse und damit auch der Gasproduktion führen, was als "Fermenterabsturz" bezeichnet wird. Um eine Eindickung zu verhindern, kann durch Zugabe flüssiger Substanzen, wie zum Beispiel Wasser oder Gülle eine Verflüssigung des Fermenterinhaltes erreicht werden. Die Problematik dabei liegt bei den gesetzlichen Rahmenbedingungen (EEG), die bei Erhalt des Trockenfermentations-Bonus eine Zugabe von Substanzen, die einen geringeren Trockensubstanzanteil als durchschnittlich 30 % aufweisen, nicht gestatten. Eine Verflüssigung des Fermentermaterials durch zusätzliche Flüssigkeitszufuhr kann daher selten angewendet werden.

Bedenkt man zusätzlich zu den oben genannten Erwägungen die Knappheit der Ressource Süßwasser, so scheidet eine Verflüssigung des Fermenterinhalts durch Zugabe von Wasser aus. Gülle als Verflüssigungsstoff ist zum einen wegen des technischen Aufwandes mit hohen Kosten verbunden, zum anderen nicht überall verfügbar und außerdem von wechselnder Qualität und Güte. Außerdem ist beim Einsatz von Gülle länderspezifisch nach dem Fermentationsprozess eine Hygienisierungsstufe aus rechtlichen Gründen erforderlich, bevor der Gärrest wieder auf das Feld ausgebracht werden kann.

WO 2006/056819 offenbart ein Verfahren zur Steigerung der Produktion von Biogas aus Biomasse, dadurch gekennzeichnet, dass der Biomasse ein thermophiler, acetogener und Wasserstoff produzierender Mikroorganismus zugegeben wird, beispielsweise ein Mikroorganismus des Genus *Thermotoga, Thermococcus, Pyrococcus, Clostridium, Ruminococcus* oder *Caldicellulosiruptor.*

Nielsen H. B. et al. (2007), Biotechnology and Bioengineering 97 (6), 1638-1643, beschreibt ein weiteres Verfahren zur Steigerung der Produktion von Biogas aus Biomasse, dadurch gekennzeichnet, dass der Biomasse ein Mikroorganismus des Genus *Caldicellulosiruptor* oder *Dictyoglomus* zugesetzt wird.

Aus CA 2258254 A1 ist eine Vorrichtung zur industriellen Aufbereitung von Abwässern und Klärschlamm bekannt. Durch mesophile anaerobe Fermentation organischer Materialien wird dabei Biogas hergestellt.

WO 2007/052306 A offenbart ein dreistufiges Verfahren zur Herstellung von Biogas aus Biomasse, in welchem in der ersten Stufe (Hydrolyse) ein Mikroorganismus, beispielsweise *Bacillus subtilis* oder *Clostridium propionicum,* zugegeben wird, um die Hydrolyse zu beschleunigen.

WO 2006/119052 A offenbart ein Verfahren zur Herstellung von Wasserstoff aus Biomasse durch anaerobe Fermentation, in welchem der Biomasse nach Sterilisation ein Bakterium des Genus *Clostridium* zugesetzt wird.

Es besteht daher weiterhin ein Bedarf an Verfahren zur Erzeugung von Biogas, die eine gegenüber dem Stand der Technik erhöhte Ausbeute an Biogas ermöglichen.

### Definitionen

Unter dem Begriff "Biokraftstoff" wird ein flüssiger oder gasförmiger Kraftstoff bzw. Energieträger verstanden, der aus Biomasse hergestellt wird. Biokraftstoffe kommen für den Betrieb von Verbrennungsmotoren sowohl für mobile (z.B. Kraftfahrzeuge) als auch stationäre (z.B. Erzeugung von Elektro- und Wärmeengergie in einem Blockheizkraftwerk) Anwendungen zum Einsatz. Beispiele für Biokraftstoffe sind Biodiesel, Bioethanol, Biomethanol, Biokerosin, Biowasserstoff oder Biogas.

Unter dem Begriff "Bioethanol" wird Ethanol verstanden, der durch alkoholische Gärung aus Biomasse als nachwachsendem Kohlenstoffträger oder biologisch abbaubaren Anteilen von Abfällen hergestellt wurde. Er dient in verschiedenen Konzentrationen als Zusatz zu Mineralölkraftstoffen wie Biodiesel oder Biokraftstoff.

Unter dem Begriff "Biogas" wird im Rahmen der vorliegenden Erfindung das gasförmige Produkt des anaeroben biologischen Abbaus organischer Substrate verstanden. Es enthält in der Regel ca. 45-70 % Methan, 30-55 % Kohlendioxid, sowie geringe Mengen an Stickstoff, Schwefelwasserstoff und anderen Gasen.

Die Begriffe "Fermentation" oder "Fermentierung" umfassen im Sinne der vorliegenden Erfindung sowohl anaerobe wie auch aerobe Stoffwechselprozesse, die unter Einwirkung von Mikroorganismen in einem technischen Prozess aus dem zugeführten Substrat zur Erzeugung eines Produktes, z.B. Biogas führen. Eine Abgrenzung zu dem Begriff "Gärung" ist insofern gegeben, dass es sich bei diesem ausschließlich um anaerobe Prozesse handelt.

Unter einem "Fermenter" wird im Rahmen der vorliegenden Erfindung der Behälter verstanden, in dem der mikrobiologische Abbau des Substrates bei gleichzeitiger Biogasbildung stattfindet. Die Begriffe "Reaktor", "Gärbehälter" und "Faulbehälter" werden synonym verwendet.

Unter den Begriffen "Gärsubstrat" oder "Substrat" wird im Rahmen der vorliegenden Erfindung organisches und biologisch abbaubares Material verstanden, das dem Fermenter zur Fermentation zugesetzt wird. Substrate können nachwachsende Rohstoffe, Wirtschaftsdünger, Substrate aus der weiterverarbeitenden Agrarindustrie, kommunale organische Reststoffe, Schlachtrückstände oder Grünschnitt sein. Beispiele für solche Substrate sind Maissilage, Roggensilage, Zuckerrübenschnitzel, Melasse, Grassilage, Rinder- oder Schweinegülle, Rinder-, Schweine-, Hühner- oder Pferdemist, Biertreber, Apfel-, Obst- oder Rebentrester, Getreide-, Kartoffel- oder Obstschlempe. Organischer Abfall aus Biotonne, Speisereste, Marktabfälle, Fett aus Fettabscheidern, Panseninhalt, Schweinemageninhalt. Die Begriffe "Gärsubstrat" und "Substrat" werden synonym verwendet.

Unter dem Begriff "Gärrückstand" oder "Gärgut" wird der Rückstand der Biogasgewinnung verstanden, der den Fermenter verlässt und häufig in einem eigenen Behälter gelagert wird.

Unter "Raumbelastung" wird im Rahmen der vorliegenden Erfindung die pro Tag und Kubikmeter (m³) Arbeitsvolumen dem Fermenter zugeführte Menge an organischer Trockensubstanz (oTS) in kg verstanden.

Unter "organischer Trockensubstanz" (oTS) wird im Rahmen der vorliegenden Erfindung der wasserfreie organische Anteil eines Stoffgemisches nach Entfernung der anorganischen Anteile und Trocknung bei 105 °C verstanden. In der Regel wird der Trockensubstanzanteil in % des Substrats angegeben.

Unter dem Begriff "Verweilzeit" wird im Rahmen der vorliegenden Erfindung die durchschnittliche Aufenthaltszeit des Substrates im Fermenter verstanden.

Unter dem Begriff "spezifische Biogasausbeute" oder "spezifische Methanausbeute" wird im Rahmen der vorliegenden Erfindung die Menge an erzeugtem Biogas oder Methan (angegeben in Normkubikmeter Nm³ erzeugtem Gas) dividiert durch die Menge (in der Regel pro Tonne t) an eingesetzter organischer Trockensubstanz oder Substrat verstanden.

Unter dem Begriff "Raumzeitausbeute" wird die erzeugte Menge an Biogas in Normliter (NI) normiert auf einen Liter Gärvolumen angegeben. Diese Meßgröße dient zur besseren Vergleichbarkeit des Gasertrags verschiedener Anlagen.

Von dem Begriff "Nukleotidsequenz" wird sowohl die DNA-Sequenz als auch die korrespondierende RNA-Sequenz umfasst. In der Erfindung angegebene RNA-Sequenzen unter der Verwendung der Basen A, U, C, G beziehen sich beispielsweise ebenso auf die korrespondierenden DNA-Sequenzen unter Verwendung der Basen A, T, C, G und umgekehrt.

Die Bestimmung der Nukleotid-Sequenz eines Mikroorganismus erfolgt mit Hilfe eines standardisierten Prozesses, durch den die einzelnen Nukleotide der DNA oder RNA des Mikroorganismus mit hoher Genauigkeit nachgewiesen werden können. Trotz der hohen Genauigkeit der Sequenzierungsverfahren können in einer Sequenz immer wieder einzelne Positionen vorliegen, für die die Bestimmung des an der jeweiligen Position vorliegenden Nukleotids nicht mit hinreichender Genauigkeit möglich war. In solchen Fällen ist im Rahmen der vorliegenden Erfindung in der Nukleotidsequenz der Buchstabe "N" angegeben. Wird nachfolgend in einer Nukelotidsequenz der Buchstabe "N" verwendet, so steht dies als Kürzel für jedes denkbare Nukleotid, also für A, U, G oder C im Falle einer Ribonukleinsäure oder für A, T, G oder C im Falle einer Desoxyribonukleinsäure.

Der Begriff "Nukleotidmutation" wie hier verwendet, bedeutet eine Veränderung der Ausgangsnukleotidsequenz. Dabei können einzelne Nukleotide oder mehrere, direkt aufeinander folgende oder durch nicht veränderte Nukleotide unterbrochene Nukleotidsequenzen entfernt (Deletion), hinzugefügt (Insertion oder Addition) oder durch andere ersetzt (Substitution) werden. Der Begriff schließt auch jede mögliche Kombination der einzelnen genannten Veränderungen ein.

Der Begriff "Deletion" wie hier verwendet bedeutet das Entfernen von 1, 2 oder mehr Nukleotiden aus der jeweiligen Ausgangssequenz.

Der Begriff "Insertion" oder "Addition" wie hier verwendet bedeutet das Hinzufügen von 1, 2 oder mehr Nukleotiden zu der jeweiligen Ausgangssequenz.

Der Begriff "Substitution" wie hier verwendet bedeutet den Austausch eines an einer bestimmten Position vorhandenen Nukleotids durch einen anderes.

Unter dem Begriff "Mikroorganismus" werden mikroskopisch kleine Lebewesen verstanden, die in der Regel Einzeller sind, aber auch Mehrzeller sein können. Beispiele für Mikroorganismen sind Bakterien, mikroskopische Algen, Pilze oder Protozoen.

Unter den Bezeichnungen "Gattung" von Mikroorganismen, "Art" von Mikroorganismen und "Stamm" von Mikroorganismen wird im Rahmen der vorliegenden Erfindung die entsprechende grundlegende Kategorie der biologischen Taxonomie, insbesondere der phylogenetischen Klassifikation verstanden. Gattungen, Arten und Stämme von Mikroorganismen werden u.a. anhand ihrer RNA-Sequenzen identifiziert und unterschieden. Unter eine bestimmte Gattung, eine bestimmte Art bzw. einen bestimmten Stamm fallen dabei nicht nur Mikroorganismen mit einer ganz bestimmten RNA-Sequenz, sondern auch bis zu einem gewissen Umfang deren genetische Varianten, wobei die genetische Varianz in der Reihe Stamm, Art, Gattung zunimmt.

Die Definition einer "Art" eines Bakteriens befindet sich im wissenschaftlichen Wandel und ist weder abgeschlossen noch sind die verschiedenen Konzepte unumstritten. In der vorliegenden Erfindung wird unter "Art" das Artkonzept verstanden, das sich auf genomische und phylogenetische Analysen bezieht und in dem Review von Staley (Philos. Trans. R. Soc. Lond. B. Biol. Sci., 2006, 361, 1899-1909) beschrieben ist. Weitestgehend anerkannt ist, dass zwei bakterielle Spezies, die mehr als 70 % DNA-DNA-Hydridisierung oder mehr als 94 % durchschnittliche Nukleotididentität (ANI, average nucleotide identity) aufweisen, derselben Art angehören bzw. zwei Spezies, die weniger als 97 % Identität der 16S rRNA aufweisen, zwei unterschiedlichen Arten zuzuordnen sind.

Unter dem Begriff "Kultur" versteht sich in diesem Zusammenhang eine Ansammlung von Mikroorganismen unter geschaffenen Bedingungen, die das Wachstum oder zumindest das Überleben der Mikroorganismen gewährleisten. Für Bakterien sind das z.B. Anreicherungskulturen oder Reinkulturen, Flüssigkulturen ebenso wie Kulturen auf festen Medien wie Nährböden, aber auch Dauerkulturen wie tiefgekühlte Glycerinkulturen, immobilisierte Kulturen wie z.B. Gelkulturen oder hochkonzentrierte Kulturen wie Zellpellets.

Unter dem Begriff "Reinkultur" eines Mikroorganismus wird die Nachkommenschaft einer einzelnen Zelle verstanden. Diese wird durch einen mehrstufigen Prozess aus einem Gemisch verschiedener Mikroorganismen isoliert. Der mehrstufige Prozess umfasst die Abtrennung einer einzelnen Zelle aus einer Zellpopulation und erfordert, dass auch die aus der Zelle durch Wachstum und Zellteilung hervorgehende Kolonie von anderen Einzelzellen oder Kolonien getrennt bleibt. Durch eine sorgfältige Abtrennung einer Kolonie, erneutes Suspendieren in Flüssigkeit und wiederholtes Ausstreichen können gezielt Reinkulturen von Mikroorganismen gewonnen werden. Die Isolierung einer Reinkultur kann auch in flüssigen Nährmedien erfolgen, sofern der gewünschte Organismus im Ausgangsmaterial zahlenmäßig überwiegt. Durch serienmäßige Verdünnung der Suspension in der Nährlösung lässt es sich schließlich erreichen, dass sich in der letzten Verdünnungsstufe nur noch eine Zelle befindet. Diese Zelle stellt dann die Basis für eine Reinkultur dar. Die Erläuterung des Begriffs "Reinkultur" und Verfahren zur Erzeugung derselben sind in "Allgemeine Mikrobiologie" von Hans G. Schlegel, 7. überarbeitete Auflage, 1992, Georg Thieme Verlag, S. 205 aufgeführt, worauf hiermit vollinhaltlich Bezug genommen wird.

Unter dem Begriff "Mischkultur" wird ein Gemisch verschiedener Mikroorganismen verstanden. Natürliche Populationen von Mikroorganismen sind in der Regel Mischkulturen. Mischkulturen können jedoch auch künstlich hergestellt werden z.B. durch die Vereinigung von mehreren Reinkulturen.

### Darstellung der Erfindung

Die Aufgabe der Erfindung besteht darin, ein Verfahren zur Erzeugung von Biogas aus Biomasse bereitzustellen, das eine gegenüber dem Stand der Technik erhöhte Ausbeute an Biogas ermöglicht.

Diese Aufgabe wird erfindungsgemäß durch das Verfahren zur Behandlung von Biomasse gemäß Anspruch 1 gelöst. Weitere vorteilhafte Details, Aspekte und Ausgestaltungen der vorliegenden Erfindung ergeben sich aus den abhängigen Ansprüchen, der Beschreibung und den Beispielen.

Die vorliegende Erfindung stellt ein Verfahren zur Erzeugung von Biogas aus Biomasse zur Verfügung. Der Biomasse wird ein Mikroorganismus der Art *Clostridium sporosphaeroides* zugesetzt.

Überraschenderweise hat sich gezeigt, dass durch die Zugabe von Mikroorganismen der Art *Clostridium sporosphaeroides* zum Gärsubstrat sowohl die Raumbelastung des Fermenters gesteigert werden kann als auch die Menge an gebildetem Biogas deutlich erhöht wird. Wie in experimentellen Untersuchungen gezeigt werden konnte, bewirkt die Zugabe eines Mikroorganismus der Art *Clostridium sporosphaeroides* eine Steigerung der Raumbelastung eines Fermenters bis hin zu mehr als 50 %, ohne dass eine Instabilität des Fermentationsprozesses eintreten würde. Parallel zu der erhöhten Raumbelastung wird die Menge an gebildetem Biogas deutlich gesteigert. Zudem steigt die spezifische Ausbeute an Biogas an, da deutlich mehr der organischen Trockensubstanz abgebaut wird als bei fehlender Zugabe von Mikroorganismen der Art *Clostridium sporosphaeroides.* Durch den erhöhten Abbaugrad kann eine deutlich erhöhte spezifische Gasausbeute bei einer verbesserten Substratausnutzung erzielt werden. Außerdem kann durch die Zugabe von Mikroorganismen der Art *Clostridium sporosphaeroides* die Verweilzeit des Gärsubstrats im Fermenter bei konstanter Gasausbeute deutlich verkürzt werden, wodurch auch die Erhöhung der Raumbelastung möglich wird. Der Einsatz von Mikroorganismen der Art *Clostridium sporosphaeroides* führt daher zu einer dramatischen Verbesserung von Effizienz und Wirkungsgrad von Biogasanlagen.

Eine mögliche Erklärung der positiven Eigenschaften der Mikroorganismen der Art *Clostridium sporosphaeroides* liegt in einer durch deren Stoffwechselaktivität gesteigerten Hydrolyserate, so dass als Ergebnis der Stoffwechselaktivität ein Teil der vorliegenden schwer abbaubaren organischen Substanzen (z.B. Cellulose, Hemicellulose) in lösliche Säuren und CO₂ übergeht. Cellulose ist in Wasser und wässrigen Lösungen unlöslich, daher führt ein Abbau der Cellulose im Fermentationsprozess zu einer Verflüssigung. Die Rühr- und Pumpfähigkeit des Materials bleibt erhalten, da der Trockensubstanzgehalt nicht weiter ansteigt. Dadurch wird eine konstante Durchmischung des Materials ermöglicht und das Gas kann aus dem Prozess besser abgeführt werden.

Durch die erhöhte Abbau- bzw. Stoffwechselleistung von Mikroorganismen der Art *Clostridium sporosphaeroides* in der Hydrolyse-Stufe wird eine Verflüssigung des Fermentermaterials herbeigeführt, ohne dass dabei eine Versäuerung, bzw. eine Verschlechterung des Methangehaltes des entstehenden Biogases zu beobachten ist. Die Raumbelastung wird deutlich erhöht und die Stabilität des Prozesses verbessert.

Durch die erfindungsgemäße Zugabe von Mikroorganismen der Art *Clostridium sporosphaeroides* wird also ein Verfahren bereitgestellt, das eine erhöhte Stabilität des Fermentationsprozesses gewährleistet und bei dem es zu einer Verflüssigung des Gärsubstrates kommt.

Gemäß der vorliegenden Erfindung wird ein Mikroorganismus der Art *Clostridium sporosphaeroides* in Form einer Kultur von Mikroorganismen zugesetzt, die überwiegend aus einem Mikroorganismus der Art *Clostridium sporosphaeroides* besteht. In Gärsubstraten von Biogasanlagen konnten Mikroorganismen der Art *Clostridium sporosphaeroides* nur in geringsten Spuren von weniger als 10⁻⁴ % Anteil an der gesamten Anzahl von anwesenden Mikroorganismen nachgewiesen werden. Da für die Zugabe der Mikroorganismen die Menge an aus ihrem natürlichen Vorkommen isolierten Mikroorganismen in der Regel nicht ausreicht, wird üblicherweise eine Vermehrung in Form einer Kultur vorgenommen. In der Praxis zeigt sich, dass die Zugabe der Mikroorganismen zu dem Gärsubstrat eines Fermenters am einfachsten direkt in Form einer Kultur von Mikroorganismen vorgenommen wird.

Die Zugabe der Kultur von *Clostridium sporosphaeroides* kann in Form einer Kultursuspension, in Form trockener, gefriergetrockneter oder feuchter Zellpellets oder auch in Form von Sporensuspensionen, Sporenpräparaten oder trockener, gefriergetrockneter oder feuchter Sporenpellets vorgenommen werden.

Da die bereits angesprochenen verschiedenen positiven Effekte auf den Gärprozess mit der Mikroorganismenart *Clostridium sporosphaeroides* verbunden sind, sollte diese Art von Mikroorganismen in der zugegebenen Kultur in einer das natürliche Vorkommen übersteigenden Menge anwesend sein. Selbstverständlich können Mischkulturen in beliebiger Zusammensetzung für die Zugabe verwendet werden. Voraussetzung ist lediglich, dass die Art *Clostridium sporosphaeroides* in einer gegenüber dem natürlichen Vorkommen angereicherten Menge anwesend ist.

Bevorzugt sind Mischkulturen von Mikroorganismen der Art *Clostridium sporosphaeroides* mit Mikroorganismen der Arten *Clostridium sartagoformum* und/oder *Paenibacillus macerans.* Besonders bevorzugt sind Mischkulturen von Mikroorganismen des Stammes *Clostridium sporosphaeroides* SBG3 (DSM 22577) mit Mikroorganismen der Stämme *Clostridium sartagoformum* SBG1a (DSM 22578) und/oder *Paenibacillus macerans* SBG2, (DSM 22 569). Mikroorganismen der Arten *Clostridium sartagoformum* und *Paenibacillus macerans* weisen in Bezug auf die Behandlung von Biomasse ähnliche Eigenschaften wie Mikroorganismen der Art *Clostridium sporosphaeroides* auf, weshalb sie für den Einsatz bei der Behandlung von Biomasse prädestiniert sind. Mikroorganismen der Arten *Clostridium sartagoformum* und *Paenibacillus macerans* können daher ebenfalls in den hier beschriebenen Verfahren und Anwendungen zur Behandlung von Biomasse eingesetzt werden.

Mikroorganismen der Art *Clostridium sporosphaeroides* werden bevorzugt in Form von Kulturen von Mikroorganismen dem Gärsubstrat zugesetzt, wobei die Kulturen von Mikroorganismen überwiegend aus Mikroorganismen der Art *Clostridium sporosphaeroides* bestehen. Wird neben der Bestimmung der Anzahl an Mikroorganismen der Art *Clostridium sporosphaeroides* auch die Gesamtzahl an Mikroorganismen bestimmt, so kann der Anteil an Mikroorganismen der Art *Clostridium sporosphaeroides* in der Kultur in Prozent angegeben werden. Mikroorganismen der Art *Clostridium sporosphaeroides* sind in einer Mischkultur dann die überwiegend anwesende Art von Mikroorganismen, wenn sie den höchsten prozentualen Anteil der verschiedenen in der Mischkultur anwesenden Arten von Mikroorganismen aufweisen.

Die Zusammensetzung der mikrobiellen Populationen in den verschiedenen Gärsubstraten sowie die Entwicklung der Organismenzusammensetzung während des Fermentationsprozesses ist größtenteils unbekannt, jedoch sehr variabel und unterliegt einem komplizierten dynamischen Prozess, der auch durch die jeweiligen Prozessbedingungen beeinflusst wird. Zur Bestimmung der mikrobiellen Zusammensetzung sowie der Gesamtzahl von Mikroorganismen in einem Gärsubstrat wie auch der Bestimmung der Anteile verschiedener Arten von Mikroorganismen in dem Gärsubstrat sind dem Fachmann verschiedene Methoden bekannt, die beispielsweise in dem Übersichtsartikel von Amann et al. (Microbiol. Review. 59, 143-169, 1995) beschrieben sind.

Eine bevorzugte Methode zur Bestimmung der Mikroorganismenzusammensetzung unabhängig von einer vorherigen Kultivierung der Mikroorganismen ist zum Beispiel die Erstellung einer rDNA Klonbibliothek (z.B. auf der Basis von 16S rRNA) nach Nukleinsäureextraktion und PCR, die anschließend sequenziert werden kann. Mit Hilfe dieser Klonbibliothek kann beispielsweise durch *in situ* Hybridisierung mit spezifischen Fluoreszenz-markierten Oligonukleotidsonden die Zusammensetzung der mikrobiellen Population im Gärsubstrat ermittelt werden. Geeignete rRNAbasierende Oligonukleotidsonden sind aus dem oben erwähnten Review bekannt oder können beispielsweise mittels probeBase (Loy et al., 2003, Nucleic Acids Res. 31, 514-516. Loy et al., 2007. Nucleic Acids Res. 35: D800-D804) oder dem ARB Programmpaket (Ludwig et al., Nucleic Acids Research. 2004, 32, 1363-1371) gefunden werden. Eine quantitative Bestimmung des Anteils einzelner Mikroorganismen an der Gesamtpopulation kann in geeigneter Weise mit den Methoden quantitativer Dot Blot, *in situ* Hybridisierung oder der Hybridisierung von ganzen Zellen (whole cell hybrisization) durchgeführt werden.

Gemäß der vorliegenden Erfindung macht der Mikroorganismus *Clostridium sporosphaeroides* zumindest 1% der Gesamtzahl an in der zu dem Gärsubstrat zugegebenen Kultur vorhandenen Mikroorganismen aus.

Gemäß weiteren bevorzugten Ausführungsformen macht der Mikroorganismus *Clostridium sporosphaeroides* zumindest 10 % der Gesamtzahl an in der Kultur vorhandenen Mikroorganismen aus,

Gemäß einer ganz besonders bevorzugten Ausführungsformen wird eine Reinkultur eines Mikroorganismus der Art *Clostridium sporosphaeroides* zugesetzt. Die Reinkultur ist biochemisch durch spezifische Stoffwechselprozesse und -aktivitäten, sowie durch spezielle Wachstumsbedingungen gekennzeichnet. Aufgrund der spezifischen Stoffwechselprozesse und -aktivitäten kann die Zugabe einer Reinkultur eines fermentativen Mikroorganismus in besonderem Maße zu einer verbesserten Kontrolle des komplexen Biogaserzeugungsprozesses beitragen. Gemäß einer weiteren bevorzugten Ausführungsform der vorliegenden Erfindung wird ein Mikroorganismus der Art *Clostridium sporosphaeroides* als Bestandteil zumindest einer immobilisierten Kultur von Mikroorganismen zugesetzt. Da für die Zugabe der Mikroorganismen die Menge an aus ihrem natürlichen Vorkommen isolierten Mikroorganismen nicht ausreicht, wird üblicherweise eine Vermehrung in Form einer Kultur vorgenommen. In der Praxis zeigt sich, dass die Zugabe der Mikroorganismen zu dem Gärsubstrat eines Fermenters am einfachsten in Form einer immobilisierten Kultur von Mikroorganismen vorgenommen wird.

Da die bereits angesprochenen verschiedenen positiven Effekte auf den Gärprozess mit der Mikroorganismenart *Clostridium sporosphaeroides* verbunden sind, sollte diese Art von Mikroorganismen in der zugegebenen immobilisierten Kultur in einer im Vergleich zum natürlichen Vorkommen angereicherten Menge anwesend sein. Selbstverständlich können immobilisierte Mischkulturen in beliebiger Zusammensetzung für die Zugabe verwendet werden. Voraussetzung ist lediglich, dass Mikroorganismen der Art *Clostridium sporosphaeroides* in einer Menge enthalten sind, die deren natürliches Vorkommen übersteigt.

Gemäß der vorliegenden Erfindung macht der Mikroorganismus der Art *Clostridium sporosphaeroides* zumindest 1 % der Gesamtzahl an in der immobilisierten Kultur vorhandenen Mikroorganismen aus.

Gemäß weiteren bevorzugten Ausführungsformen macht der Mikroorganismus der Art *Clostridium sporosphaeroides* zumindest 10 % der Gesamtzahl an in der immobilisierten Kultur vorhandenen Mikroorganismen aus,

Gemäß einer ganz besonders bevorzugten Ausführungsformen wird zumindest eine immobilisierte Reinkultur eines Mikroorganismus der Art *Clostridium sporosphaeroides* zugesetzt.

Als Trägermaterialien, auf denen der Mikroorganismus *Clostridium sporosphaeroides* immobilisiert wird, können natürliche oder synthetische Polymere eingesetzt werden. Bevorzugt werden gelbildende Polymere verwendet. Diese haben den Vorteil, dass Bakterien innerhalb der Gelstruktur aufgenommen bzw. eingelagert werden können. Bevorzugt werden solche Materialien eingesetzt, die sich in Wasser langsam auflösen bzw. abgebaut werden, so dass die Freisetzung des Mikroorganismus *Clostridium sporosphaeroides* über einen längeren Zeitraum hinweg erfolgt.

Beispiele für geeignete Polymere sind Polyanillin, Polypyrrol, Polyvinylpyrolidon, Polystyrol, Polyvinylchlorid, Polyvinylalkohol, Polyethylen, Polypropylen, Epoxidharze, Polyethylenimine, Polysaccharide wie Agarose, Alginat oder Cellulose, Ethylcellulose, Methylcellulose, Carboxymethylethylcellulose, Celluloseacetate, Alkali-Cellulosesulfat, Copolymere aus Polystyrol und Maleinsäureanhydrid, Copolymere aus Styrol und Methylmethacrylat, Polystyrolsulfonat, Polyacrylate und Polymethacrylate, Polycarbonate, Polyester, Silikone, Cellulosephthalat, Proteine wie Gelatine, Gummi arabicum, Albumin oder Fibrinogen, Gemische aus Gelatine und Wasserglas, Gelatine und Polyphosphat, Gelatine und Copolymere aus Maleinsäureanhydrid und Methylvinylether, Celluloseacetatbutyrat, Chitosan, Polydialkyldimethylammonium-chlorid, Mischungen aus Polyacrylsäuren und Polydiallyldimethylammoniumchlorid sowie deren Gemische. Das Polymermaterial kann auch mit Hilfe üblicher Vernetzer wie Glutaraldehyd, Harnstoff/Formaldehydharzen oder Tanninverbindungen vernetzt werden.

Alginate als Immobilisate erweisen sich als besonders vorteilhaft, da sie zum einen keinen negativen Einfluss auf die Aktivität des Mikroorganismus *Clostridium sporosphaeroides* nehmen und da sie zum anderen durch Mikroorganismen langsam abgebaut werden. Durch den langsamen Abbau der Alginat-Immobilisate werden nach und nach die eingeschlossenen Mikroorganismen der Art *Clostridium sporosphaeroides* freigesetzt.

Für die Immobilisierung werden die Mikroorganismen mit einem Polymergel vermengt und dann in einer geeigneten Härterlösung gehärtet. Dazu werden sie zunächst mit einer Gellösung vermischt und anschließend in eine Härterlösung aus geeigneter Höhe getropft. Die genauen Vorgehensweisen zur Immobilisierung sind dem Fachmann bekannt.

Gemäß einer weiteren bevorzugten Ausführungsform wird zeitnah zur Zugabe der nachfolgend beschriebenen Mikroorganismen zusätzliche Biomasse in den Fermentierungsreaktor gegeben. Eine zeitnahe Zugabe von zusätzlicher Biomasse kann innerhalb einer Zeitspanne von 1 Sekunde bis hin zu 3 Tagen nach Zugabe von Mikroorganismen erfolgen oder sie kann gleichzeitig mit der Zugabe von Mikroorganismen erfolgen. Dabei kann die Raumbelastung im Fermentationsreaktor durch kontinuierliche Zugabe von neuem Substrat kontinuierlich gesteigert oder in etwa konstant gehalten werden, wobei die Fermentierung bei allen Raumbelastungen, bevorzugt bei einer Raumbelastung von ≥ 0.5 kg organischer Trockensubstanz pro m³ und Tag [kgoTS/m³d], weiter bevorzugt bei einer Raumbelastung von ≥ 4.0 kgoTS/m³d und besonders bevorzugt bei einer Raumbelastung von ≥ 8.0 kgoTS/m³d durchgeführt werden kann, was im Vergleich zum gegenwärtigen Stand der Technik einer Erhöhung der Raumbelastung um mehr als das Doppelte entspricht.

Das verwendete Gärsubstrat kann insbesondere auch einen hohen Anteil an festen Bestandteilen aufweisen. Durch die Zugabe eines hydrolytisch aktiven, fermentativen Mikroorganismus der Art *Clostridium sporosphaeroides* werden diese festen Bestandteile zumindest teilweise verflüssigt. Durch die erzielte Verflüssigung des Gärsubstrats aufgrund der Zugabe des Mikroorganismus *Clostridium sporosphaeroides* kann einem Eindicken des Fermentermaterials vorgebeugt und gezielt entgegengewirkt werden. Ein weiterer Flüssigkeitseintrag in das Gärsubstrat in Form von Wasser oder Gülle während der Fermentierung kann vermieden werden. Somit besteht ein weiterer Vorteil in der Schonung der Ressource Süßwasser. Ebenfalls von Vorteil ist der so erzielte Erhalt der Rühr- und Pumpfähigkeit des Substrats. Dadurch werden Rührwerke und Pumpen geschont und für den Rührvorgang ist deutlich weniger Energie erforderlich.

Gemäß einer weiteren Ausführungsform erfolgt die Erzeugung von Biogas aus Biomasse unter konstanter Durchmischung des Gärsubstrats. Durch die konstante Durchmischung des Gärsubstrats können die Kulturen von *Clostridium sporosphaeroides* besser im Gärsubstrat verteilt werden. Im Falle der Biogasherstellung kann außerdem das gebildete Biogas besser aus dem Fermentationsprozess abgeführt werden.

Die konstante Durchmischung des Gärsubstrats führt zudem zu einer gleichmäßigen Wärmeverteilung im Fermentationsreaktor. Messungen der Temperatur im Fermentationsreaktor, die in periodischen Abständen, aber auch kontinuierlich durchgeführt wurden, ergaben, dass das Gärsubstrat in einem Temperaturbereich von 20 °C bis 80 °C, bevorzugt bei etwa 35 °C bis 60 °C, besonders bevorzugt bei 40 °C bis 50 °C effizient fermentiert wird. Diese Temperaturbereiche werden daher bevorzugt. Neben der Hydrolyse erfolgt insbesondere die letzte Stufe des Fermentationsprozesses, nämlich die Bildung von Methan durch methanogene Mikroorganismen, besonders effizient bei erhöhten Temperaturen. Für mehrstufige Biogasanlagen ist es auch sinnvoll, die unterschiedlichen Reaktoren bei unterschiedlichen Temperaturen zu betreiben, z.B. die erste Stufe unter mesophilen Bedingungen und die nachgeordneten Stufen, insbesondere die Methanogenese, bei thermophilen Bedingungen. Geeignete Bedingungen hierfür sind aus dem Stand der Technik bekannt (z.B. DE 10 2005 012367 A1).

Sämtliche Ausführungsformen der vorliegenden Erfindung sind nicht auf einstufige Verfahren zur Herstellung von Biogas beschränkt. Der Einsatz von Mikroorganismen der Art *Clostridium sporosphaeroides* kann auch in zwei- oder mehrstufigen Verfahren erfolgen.

Gemäß einer weiteren Ausführungsform werden Gärsubstrat und ein Mikroorganismus der Art *Clostridium sporosphaeroides* kontinuierlich zugegeben. Der kontinuierliche Betrieb eines Fermentationsreaktors soll bei einer stabilen mikrobiellen Biozönose zu einer kontinuierlichen Produktion von Biogas führen, wobei das Aussetzen der Substratzugabe zur Fermentation infolge einer Prozessstörung vermindert werden soll.

Ebenfalls ist die Ausführung dieses Fermentationsverfahrens und der damit zusammenhängenden Prozesse auch in einem diskontinuierlichen Betrieb, beispielsweise "Batch"-Fermentierung denkbar. So kann dem Gärsubstrat gemäß einer weiteren Ausführungsform während der Fermentierung der Mikroorganismus der Art *Clostridium sporosphaeroides* beispielsweise in regelmäßigen Abständen zugegeben werden. Die Zugabe des Mikroorganismus der Art *Clostridium sporosphaeroides* in regelmäßigen Abständen führt zu einer Steigerung der Lebendzellzahl und somit zu einem verbesserten Ablauf der fermentativen Prozesse, beispielsweise der Hydrolyse bei einer gleichzeitig verbesserten Ausnutzung des Gärsubstrats für die Fermentierung.

Gemäß einer bevorzugten Ausführungsform der vorliegenden Erfindung wird der Mikroorganismus der Art *Clostridium sporosphaeroides* in einer Menge zu dem Gärsubstrat zugegeben, so dass nach Zugabe der Anteil des Mikroorganismus der Art *Clostridium sporosphaeroides* zwischen [10⁻⁴% und 10 %] der Gesamtzahl an in dem Gärsubstrat anwesenden Mikroorganismen ausmacht. Insbesondere in Abhängigkeit von der Fermentergröße und damit in Abhängigkeit von der Menge an Gärsubstrat kann zur Erzielung der gewünschten Wirkung eine Zugabe von sehr stark unterschiedlichen Mengen an Mikroorganismen notwendig werden.

Sowohl die Bestimmung der Gesamtzahl von Mikroorganismen in dem Gärsubstrat wie auch die Bestimmung der Anteile verschiedener Arten von Mikroorganismen im Gärsubstrat stellt für den Fachmann kein Problem dar. So kann beispielsweise mit Hilfe von Fluoreszenz-markierten Oligosonden spezifisch der Anteil verschiedener Mikroorganismen in dem Gärsubstrat nach dem weiter oben beschriebenen Verfahren identifiziert werden

Besonders bevorzugt wird der Mikroorganismus der Art *Clostridium sporosphaeroides* in einer Menge zu dem Gärsubstrat zugegeben, dass nach Zugabe der Anteil des Mikroorganismus der Art *Clostridium sporosphaeroides* zwischen 10⁻³ % und 1 % der Gesamtzahl an in dem Gärsubstrat anwesenden Mikroorganismen ausmacht.

Grundsätzlich kann der Zusatz von Mikroorganismen der Art *Clostridium sporosphaeroides* zu jedem beliebigen Zeitpunkt des Fermentationsprozesses erfolgen, insbesondere können Mikroorganismen der Art *Clostridium sporosphaeroides* auch zum Animpfen von Gärsubstrat bei der erstmaligen Inbetriebnahme oder einer Wiederinbetriebnahme eines Fermenters verwendet werden. Mikroorganismen der Art *Clostridium sporosphaeroides* können in Form einer Kultur einmalig oder mehrmalig in regelmäßigen oder unregelmäßigen Abständen, bevorzugt jedoch wöchentlich oder monatlich, besonders bevorzugt täglich oder zweimal bis fünf mal pro Woche in einer geeigneten Konzentration und Menge zugegeben werden. Geeignete Konzentrationen an Mikroorganismen und zugegebenen Mengen wurden bereits genannt bzw. werden in den Ausführungsbeispielen beschrieben.

Ebenso ist es möglich Mikroorganismen der Art *Clostridium sporosphaeroides* bei Störungen des Fermentationsprozesses zur Stabilisierung der Fermentation zuzugeben. Solche Störungen können durch Überwachung bestimmter charakteristischer Parameter der Fermentierung frühzeitig erkannt werden. Charakteristische Parameter geben Auskunft über die Qualität eines ablaufenden Fermentierungsprozesses zur Herstellung von Biogas. Solche charakteristischen Parameter sind nicht nur die Menge an erzeugtem Biogas und der Methangehalt des erzeugten Biogases sondern beispielsweise auch der Wasserstoffgehalt des erzeugten Biogases, der pH-Wert des Gärsubstrats, das Redoxpotential des Gärsubstrats, der Carbonsäuregehalt des Gärsubstrats, die Anteile verschiedener Carbonsäuren im Gärsubstrat, der Wasserstoffgehalt des Gärsubstrats, der Anteil der Trockensubstanz am Gärsubstrat, der Anteil der organischen Trockensubstanz am Gärsubstrat, die Viskosität des Gärsubstrats und die Raumbelastung des Fermentierungsreaktors.

Ebenso umfasst die vorliegende Erfindung den Stamm des Mikroorganismus *Clostridium sporosphaeroides* SBG3, wie er unter der Nr. DSM 22577 hinterlegt ist. Der Mikroorganismus *Clostridium sporosphaeroides* SBG3 wurde in einer Reinkultur bei der Deutschen Sammlung von Mikroorganismen und Zellkulturen GmbH in Braunschweig gemäß dem Budapester Vertrag hinterlegt. Die Bezeichnung lautet: *Clostridium sporosphaeroides* SBG3 mit der Hinterlegungsnummer DSM 22577. Ebenso umfasst die vorliegende Erfindung ein Verfahren unter Verwendung des Mikroorganismen-stammer *Clostridium sartagoformum* SBG1a, wie er unter der Nr. DSM 22578 hinterlegt ist. Der Mikroorganismus *Clostridium sartagoformum* SBG1a wurde in einer Reinkultur bei der Deutschen Sammlung von Mikroorganismen und Zellkulturen GmbH in Braunschweig gemäß dem Budapester Vertrag hinterlegt. Die Bezeichnung lautet: *Clostridium sartagoformum* SBG1a mit der Hinterlegungsnummer DSM 22578.

Bakterien der Art *Clostridium sporosphaeroides* können mit Hilfe von dem Fachmann bekannten Methoden aus dem Gärsubstrat oder Gärrest eines Fermenters isoliert werden. Dabei wird ein geeignetes Substrat aus einem Fermenter in ein Selektionsmedium eingebracht, über längere Zeit kultiviert und schließlich einzelne Kolonien von Mikroorganismen aus dem Selektionsmedium isoliert. Nach Vervielfältigung der daraus erhaltenen mikrobiellen DNA mittels PCR können auf der Basis der DNA Mikroorganismen der Art *Clostridium sporosphaeroides* ausgewählt werden.

Bakterien *Clostridium sporosphaeroides* SBG3 wurden aus dem Gärsubstrat eines Nachgärers isoliert. Dazu wurde durch ein flüssiges Selektionsmedium Stickstoff und Kohlendioxid durchgeleitet, anschließend Na₂S zu dem Selektionsmedium zugegeben und autoklaviert (20 Min. bei 121°C). Dann wurde die aus dem Nachgärer gewonnene Biomasse in das Selektionsmedium eingebracht und für zumindest eine Woche bei einer Temperatur von mindestes 30 °C kultiviert. Eine aus dem flüssigen Selektionsmedium gewonnene Probe wurde auf ein festes Selektionsmedium aufgebracht und nachfolgend die auf dem festen Selektionsmedium gewachsenen Kolonien von Mikororganismen ausgewählt. Nach Vervielfältigung der erhaltenen mikrobiellen DNA mittels PCR konnte ein Vergleich mit bekannten DNA-Sequenzen durchgeführt werden.

Nachdem die Bakterien *Clostridium sporosphaeroides* SBG3 erfolgreich aus dem Gärsubstrat des Nachgärers isoliert worden waren, wurden diese Mikroorganismen einer Sequenzanalyse unterzogen. Es wurde eine Teilsequenz der 16S rRNA bestimmt, die dann in die entsprechende DNA-Sequenz transformiert wurde. Die DNA-Sequenz SEQ ID Nr. 1 umfasst 1409 Nukleotide. Als nächster Verwandter wurde der Mikroorganismus *Clostridium sp.* Klon 1099982248072 identifiziert, der aus einer Stuhlprobe eines Babys stammte und so mit der technischen Erzeugung von Biokraftstoffen in keiner Weise zusammenhängt (Genbank Identifikationsnummer EF434352, Länge der Sequenz 1340 Nukleotide). Ein Vergleich der Sequenzen ergab, dass bei der Sequenz von *Clostridium sp.* Klon 1099982248072 im Vergleich zu SEQ ID Nr. 1 insgesamt 46 Austausche von Nukleotiden oder Lücken vorlagen. Bei einer Länge der Sequenz von *Clostridium* sp. Klon 1099982248072 von 1340 Nukleotiden errechnet sich eine Identität von 96, 57%.

Die vorliegende Erfindung umfasst auch Mikroorganismen mit einer Nukleinsäure, die eine Nukleotidsequenz aufweist, die einen Sequenzbereich enthält, der mehr als 98,5 % Sequenzidentität mit der Nukleotidsequenz SEQ ID Nr. 1 aufweist

In bevorzugten Ausführungsformen der vorliegenden Erfindung können gegenüber der Ausgangsnukleotidsequenz SEQ ID Nr. 1 an einer Position oder an zwei Positionen oder an drei Positionen oder an vier Positionen oder an fünf Positionen oder an sechs Positionen oder an sieben Positionen oder an acht Positionen oder an neun Positionen oder an zehn Positionen oder an elf Positionen oder an zwölf Positionen oder an 13 Positionen oder an 14 Positionen oder an 15 Positionen oder an 16 Positionen oder an 17 Positionen oder an 18 Positionen oder an 19 Positionen oder an 20 Positionen oder an 21 Positionen Nukleotidmutationen vorliegen. Die Bedeutung des Begriffs "Nukleotidmutation" ist im Abschnitt "Definitionen" des vorliegenden Textes erläutert.

Die vorliegende Erfindung umfasst auch eine Kultur von Mikroorganismen geeignet zum Einsatz in einem Verfahren zur fermentativen Erzeugung von Biogas aus Biomasse, wobei in der Kultur von Mikroorganismen ein Mikroorganismus anwesend ist, der eine Nukleotidsequenz aufweist, die einen Sequenzbereich enthält, der zumindest 98,5 % Sequenzidentität mit der Nukleotidsequenz SEQ ID Nr. 1 aufweist, wobei der Mikroorganismus zumindest 1% der Gesamtzahl an in der Kultur vorhandenen Mikroorganismen ausmacht.

Gemäß weiterer bevorzugter Ausführungsformen macht der Mikroorganismus *Clostridium sporosphaeroides* zumindest 10%, der Gesamtzahl an in der Kultur vorhandenen Mikroorganismen aus. Besonders bevorzugt handelt es sich um eine Reinkultur von Mikroorganismen geeignet zum Einsatz in einem Verfahren zur fermentativen Erzeugung von Biogas aus Biomasse, wobei es sich um eine Reinkultur des Mikroorganismus *Clostridium sporosphaeroides* SBG3 wie er oben in Bezug auf seine Nukleotidsequenz charakterisiert wurde, handelt.

Besonders bevorzugt handelt es sich in den oben beschriebenen Fällen um eine immobilisierte Kultur von Mikroorganismen.

### Kurze Beschreibung der Zeichnungen

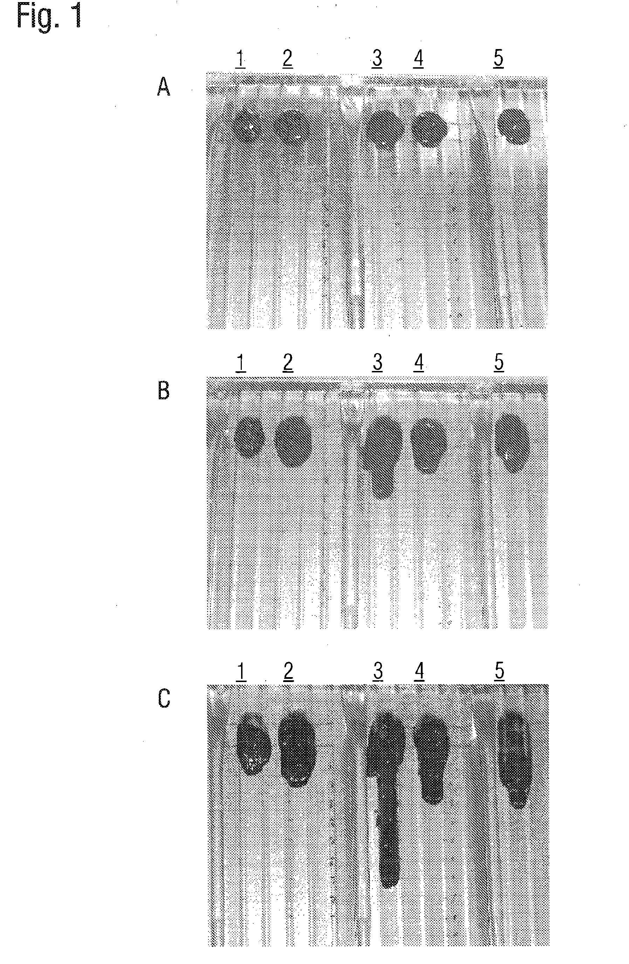

Zur Illustration der Erfindung und zur Verdeutlichung ihrer Vorzüge werden nachfolgend Ausführungsbeispiele angegeben. Die Ausführungsbeispiele sollen im Zusammenhang mit den Figuren 1 bis 4 näher erläutert werden. Es versteht sich von selbst, dass die im Zusammenhang mit den Ausführungsbeispielen gemachten Angaben die Erfindung nicht beschränken sollen. Es zeigen:
- Fig. 1: Einen Substratfließtest zur Untersuchung des Ausmaßes der Verflüssigung von Gärsubstrat;
- Fig. 2: Messergebnisse einer Fermentierung: Aufgetragen ist der Gasertrag als Raumzeitausbeute (NI/I) und die Raumbelastung des Fermenters gegen die Zeit;
- Fig. 3: Messergebnisse einer weiteren Fermentierung: Aufgetragen ist der Gasertrag als Raumzeitausbeute (NI/I) und die Raumbelastung des Fermenters gegen die Zeit;
- Fig. 4: Messergebnisse einer weiteren Fermentierung: Aufgetragen ist der Gasertrag als Raumzeitausbeute (NI/I) und die Raumbelastung des Fermenters gegen die Zeit.

### Wege zur Ausführung der Erfindung

Bakterien *Clostridium sporosphaeroides* SBG3 wurden erfolgreich aus dem Gärsubstrat eines Nachgärers isoliert. Die Hinterlegung des Organismus erfolgte in einer Reinkultur bei der Deutschen Sammlung von Mikroorganismen und Zellkulturen GmbH (DSMZ) gemäß dem Budapester Vertrag (*Clostridium sporosphaeroides* SBG3 mit der Hinterlegungsnummer DSM 22577).

Bakterien *Clostridium sartagoformum* SBG1a wurden ebenfalls erfolgreich aus dem Gärsubstrat eines Nachgärers isoliert. Die Hinterlegung des Organismus erfolgte in einer Reinkultur bei der Deutschen Sammlung von Mikroorganismen und Zellkulturen GmbH (DSMZ) gemäß dem Budapester Vertrag *(Clostridium sartagoformum* SBG1a mit der Hinterlegungsnummer DSM 22578).

Wie den folgenden Ausführungsbeispielen zu entnehmen ist, weisen Bakterien des Stammes *Clostridium sartagoformum* SBG1a sowohl in Mischkultur als auch in Reinkultur erfindungsgemäße Eigenschaften ähnlich denen zu Bakterien des Stammes *Clostridium sporosphaeroides* SBG3 auf, wie z.B. eine Erhöhung der Raumbelastung, eine Erhöhung der Gasausbeute und eine Stabilisierung des Biogasprozesses sowie die Fähigkeit zur Verflüssigung von Biomasse.

Sofern nachfolgend nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.

### Isolierung und Anreicherung der Mikroorganismen

Bakterien des Stammes *Clostridium sporosphaeroides* SBG3 wurden erfolgreich aus dem Gärsubstrat eines Nachgärers isoliert. Die Isolierung der Mikroorganismen erfolgte mithilfe eines Selektionsmediums, das Carboxymethylcellulose (CMC) als einzige Kohlenstoffquelle enthielt. Carboxymethylcellulose besitzt sehr große Ähnlichkeit mit der in Gärsubstraten von Biogasanlagen enthaltenen Cellulose und weist zudem durch die Verknüpfung der Hydroxylgruppen mit Carboxymethylgruppen (-CH₂-COOH-) eine verbesserte Löslichkeit in wässrigem Medium auf. Das zur Selektion von *Clostridium sporosphaeroides* SBG3 eingesetzte Medium (DSMZ Medium 520 plus 1 % CMC und 0,2 % Hefeextrakt) wurde mit N₂ begast, damit die Selektion unter anaeroben Bedingungen erfolgen konnte. Enthaltener Restsauerstoff wurde mit Hilfe von 0,5 g/l Na₂S reduziert.

Das Selektionsmedium wurde anschließend mit dem Überstand von Material aus einem Nachgärer (1:2000 verdünnt) beimpft. Nach einer einwöchigen Kultivierung bei 40°C zeigten sich bei mikroskopischer Analyse einzelne Stäbchen. Eine weitere Selektion der Flüssigkulturen und eine Isolierung bis hin zu Reinkulturen erfolgte durch den Ausstrich auf anaeroben Carboxymethylcellulose-Platten.

Für größere Mengen an Bakterien des Stammes *Clostridium sporosphaeroides* SBG3 (z.B. für die Zugabe in den Fermentertyp 3) erfolgte die Anzucht unter den angegebenen Bedingungen in einem 1 m³ Fermenter, der mit 100 ml Vorkultur beimpft wurde. Da das Wachstum mit einer Verdopplungszeit von etwa 2 h recht schnell erfolgt, erscheint *Clostridium sporosphaeroides* SBG3 für eine biotechnologische Anwendung besonders geeignet. Kleinere Mengen an Bakterien (z.B. für die Zugabe in den Fermentertypen 1, 2 und 4) wurden im 500 ml bis 1 l Maßstab kultiviert. Die Kultivierung für den Zusatz zu einem Fermentationsprozess erfolgte über einen Zeitraum von 1 bis 2 Tagen. Es wurden Zelldichten im Bereich von 10⁸ bis 10¹⁰ Zellen pro ml Kulturmedium erreicht. Die Bakterienzellen wurden durch Zentrifugation geerntet und in einem möglichst geringen Volumen frischen Mediums aufgenommen bevor sie im Fermentationsprozess verwendet wurden. Zur Zwischenlagerung wurden die Zellen eingefroren.

Mikroorganismen der Arten *Clostridium sartagoformum und Paenibacillus macerans* , z.B. für die Verwendung von Mischkulturen mit in etwa gleichen Anteilen an den 3 erwähnten Mikroorganismen konnten unter den gleichen Bedingungen kultiviert werden.

### DNA-Isolierung und Nukleotidsequenzbestimmung

Das Zellmaterial der gewachsenen vereinzelten Kolonien diente zur Vervielfältigung der mikrobiellen DNA mittels der Colony-PCR Methode nach einem StandardProgramm.

Zur Bestimmung der 16S rRNA Sequenz wurde aus der Zell-DNA über PCR das Gen für die 16S rRNA amplifiziert. Dafür wurden die Primer mit den Sequenzen GRGTTTGATCCTGGCTCAG und ACGGHTACCTTGTTACGACTT verwendet (angegeben in 5' → 3'Richtung; H bedeutet C, T oder A). Die als PCR-Produkte erhaltenen DNA-Stücke wurden dann in einen Klonierungsvektor kloniert (Ligation mit dem QIAGEN PCR-Cloning-Kit der Firma QIAGEN/Hilden unter Verwendung des Vektors p-Drive), in *E.coli* transformiert (gemäß QIAGEN PCR-Cloning-Handbook) und mittels Colony-PCR untersucht. Die erhaltenen Colony-PCR-Produkte wurden einer Restriktionsfragment-Längenpolymorphismen-Analyse (RFLP) zur Auswahl geeigneter Klone unterzogen. Aus den jeweiligen Klonen wurde die entsprechende Plasmid-DNA isoliert und nach der Kettenabbruchmethode (Sanger et al., 1977) sequenziert.

### Sequenzanalyse

Nach der Sequenzanalyse der Kolonien konnte die 16S rDNA bzw. ihre Transformation in die korrespondierende 16S rRNA mit dem Programmpaket ARB (Ludwig et al., Nucleic Acids Research. 2004. 32, 1363-1371) phylogenetisch analysiert und als Mikroorganismus der Art *Clostridium sporosphaeroides* klassifiziert werden. Anhand einer Analyse der klonierten 16S rDNA bzw. der korrespondierenden 16S rRNA Sequenz mittels BLAST-Programm (basic local alignment search tool) der Datenbank www.ncbi.nlm.nih.gov wurde der *Clostridium* sp. Klon 1099982248072 als nächster Verwandter ermittelt.

### Verflüssigung von cellulosehaltigem Medium

Versuche mit einer Reinkultur des hydrolytisch aktiven, fermentativen Mikroorganismus *Clostridium sporosphaeroides* SBG3 zeigten, dass der Zusatz zum stark zähflüssigen, viskosen Carboxymethylcellulose-haltigen Selektionsmedium zu einer sukzessiven Verflüssigung des Mediums bis hin zu einer wasserähnlichen Konsistenz während des Bakterienwachstums führt, was durch Schütteln des Anzuchtkolbens und visuelle Inspektion deutlich beobachtet werden kann.

### Verflüssigung von Gärsubstrat - Substratfließtest

Die Verflüssigung eines Gärsubstrates mit hohem Trockensubstanzgehalt bzw. die Aufrechterhaltung einer flüssig-breiigen Konsistenz in einem Nassgärverfahren ist essentiell, um einen reibungslosen und kostengünstigen Ablauf des technischen Prozesses zu gewährleisten. Teilweise kommt es im kontinuierlichen Betrieb auch zu einer zunehmenden "Verschleimung" des Fermenterinhalts, was sich ebenfalls negativ auf den Prozess der Biogaserzeugung auswirkt. Der Effekt, den die Zugabe von Mikroorganismen auf die Konsistenz des Gärsubstrates machte, wurde mit einem Test bestimmt, in dem das Fließverhalten von Substratproben auf einer schiefen Ebene gemessen wurde (Substratfließtest).

Ausgangsmaterial für den Fließtest war Material aus einer Biogasanlage mit einem Trockensubstanzanteil von ca. 8-12 %. Jeweils 500 ml Material aus einem Fermenter wurde in 1 l Schott-Flaschen abgefüllt und 1 Tag lang bei 40 °C inkubiert. Anschließend wurde jeweils die Bakterienzellmasse aus einer 500 ml Vorkultur (entspricht ungefähr 4 x 10¹¹ Zellen) von *Clostridium sporosphaeroides* SBG3, *Clostridium sartagoformum* SBG1a, *Paenibacillus macerans* SBG2 oder einer Mischung der drei Bakterien zu gleichen Anteilen in 1 ml Medium resuspendiert, zugegeben und für weitere 5 Tage bei 40 °C unter Schütteln inkubiert. Für die Kontrollprobe wurde nur 1 ml Medium zugegeben. Jeweils 1,3 g der Proben wurden an den Startpunkt der schiefen Ebene aus Metall gesetzt und auf einer cm-Skala die Fließgeschwindigkeit (zurückgelegte Strecke pro 10 min) der jeweiligen Proben abgelesen, die ein Maß für die Verflüssigung bzw. die Viskosität des Gärsubstrates darstellt. Die Ergebnisse des Fließtests sind in der Figur 1 dargestellt.

In der Figur 1 sind folgende Spuren gezeigt: Spur 1: Kontrolle ohne Zugabe von Mikroorganismen (Vergleichsbeispiel); Spur 2: *Clostridium sartagoformum* SBG1a (Vergleichsbeispiel); Spur 3: *Clostridium sporosphaeroides* SBG3; Spur 4: *Paenibacillus macerans* SBG2 (Vergleichsbeispiel); Spur 5: Mischung aus *Clostridium sartagoformum* SBG1a, *Clostridium sporosphaeroides* SBG3 und *Paenibacillus macerans* SBG2 zu gleichen Teilen. Figur 1A zeigt die Spuren direkt nach dem Auftrag der Proben, Figur 1B nach 2 min. und Figur 1C nach 18 min.

Es zeigte sich, dass die Viskosität des Fermenterinhalts durch die Zugabe von lebenden Bakterien des Stammes *Clostridium sporosphaeroides* SBG3 sehr stark abnahm, während die Zugabe der Organismen *Clostridium sartagoformum* SBG1a und *Paenibacillus macerans* SBG2 ebenfalls einen deutlichen, aber weniger ausgeprägten Effekt aufwiesen. Auch durch den Zusatz der Mischung der 3 Mikroorganismen konnte eine signifikante Verflüssigung des Substrates erzielt werden. In einem weiteren Versuch konnte gezeigt werden, dass die Zugabe von toten Zellen (totautoklaviert) praktisch keinen Effekt hatte. Eine visuelle Inspektion der Proben ergab auch, dass bei Zusatz lebender Zellen des Stammes *Clostridium sporosphaeroides* SBG3 der Anteil schleimiger Substanzen abnahm. Überraschend war, dass die zugesetzten Bakterien auch bei Substrat, das schon einen Fermenter und damit einen durch Mikroorganismen bewirkten Fermentierungsprozess durchlaufen hat, noch eine weitere Verflüssigung bewirkten. Dies bedeutet, dass in einem Fermentierungsprozess ohne externe Zugabe von Mikroorganismen das Substrat nicht vollständig abgebaut wird, sondern zusätzliches energetisches Potential birgt, welches durch den Zusatz der erfindungsgemäßen Mikroorganismen genutzt werden kann.

### Bestimmung der Zelldichte

Um die Zelldichte in Bezug auf lebende Bakterien zu bestimmen, wurden jeweils 10 µl Bakterienprobe (z.B. Vorkultur, Bakterienzuchtansatz, Überstand von Fermenterprobe) mit 2 µl BacLight™-Farbstoff (Invitrogen) vermischt und bei 1000 facher Vergrößerung mikroskopiert (AXIO Imager A1, Zeiss, Jena). Unter UV-Licht und Nutzung zweier verschiedener Filter kann mit dem BacLight™-System der Anteil lebender Zellen in der Probe bestimmt werden. Die Gesamtzellzahl wurde in einer Thomazählkammer ausgezählt. Der Anteil lebender Zellen aus den Vorkulturen war in der Regel höher als 90 %.

### Bestimmung des Zellanteils von Clostridium sporosphaeroides SBG3 oder Paenibacillus macerans SBG2 oder Clostridium sartagoformum SBG1a in einer Probe - "Fishing"

Der Anteil von Bakterien der Art *Clostridium sporosphaeroides* SBG3 oder *Paenibacillus macerans* SBG2 oder *Clostridium sartagoformum* SBG1a wurde durch "whole cell hybridization" nach der in Amann et al. (1995, Microbiol. Rev. 59, 143-169) beschriebenen Methode bestimmt. Als Sonde zum "Fishing" wurde für *Clostridium sporosphaeroides* SBG3 ein markiertes Oligonukleotid mit der Sequenz Cy3-CCACAGCTCTCACGCCCG (angegeben in 5' → 3'Richtung) verwendet, für *Paenibacillus macerans* SBG2 ein markiertes Oligonukleotid mit der Sequenz Cy3-GCAACCCGAACTGAGACC (angegeben in 5' → 3'Richtung) und für *Clostridium sartagoformum* SBG1a ein markiertes Oligonukleotid mit der Sequenz Cy3-CTTCATGCGAAAATGTAA (angegeben in 5' → 3'Richtung). Die Oligonukleotide trugen als Markierung jeweils am 5'-Ende den Farbstoff Indocarbocyanin (Cy3).

### Fermentertypen

Es wurden Versuche im Technikumsmaßstab in verschiedenen Fermentertypen durchgeführt. Die Fermenter wurden bei Betriebstemperaturen von ca. 40 °C betrieben. Als Substrat wurde hauptsächlich Maissilage mit einem Anteil organischer Trockensubstanz (oTS) von 30 -35 % verwendet. Um die Versorgung mit Spurenelementen für die an der Fermentation beteiligten stoffwechselaktiven Mikroorganismen sicherzustellen, wurden in der Regel kommerziell erhältliche Spurenelementemischungen zugesetzt (z.B. Novodyn®).

Fermenter Typ 1: Liegender Pfropfenstromfermenter rechteckig, Volumen 150 I, bautechnische Unterteilung des Fermenterraumes durch eingezogene Wand mit kleinflächigem Durchlass für den Substratstrom, Aufteilung des Fermenterraumes in ¼ direkt nach Substratzugabestutzen und ¾ nach Lochblende, 2-stufige Anlage.

Fermenter Typ 2: Liegender Pfropfenstromfermenter rechteckig, Volumen 150 I als 1. Stufe plus total durchmischter Rundfermenter, Volumen 200 I als 2. Stufe; Gesamtvolumen 350 I. 2-stufige Anlage mit Rezirkulation zwischen Rundfermenter und Pfropfenstromfermenter.

Fermenter Typ 3: Liegender Pfropfenstromfermenter zylindrisch, Volumen 30 m³, 1-stufige Anlage.

Fermenter Typ 4: Liegender Pfropfenstromfermenter rechteckig, Volumen 150 I, keine bautechnische Unterteilung im Fermenterraum, 1-stufige Anlage. Rezirkulation optional.

### Verteilung der Mikroorganismen innerhalb des Fermenters - "Tracer-Test"

Da die Durchmischung des zähen Substrates und damit auch sonstiger Zusätze in einem Fermenter einige Zeit dauert und außerdem von der eingesetzten Rührtechnik abhängt, wurde ein Test etabliert, mit dem der Zeitraum bis zu einer gleichmäßigen Verteilung von zugesetzten Mikroorganismen im Fermenter bestimmt werden kann. In diesem "Tracer-Test" wurde anstelle von Mikroorganismen pulverförmiges LiCl in einer Konzentration von 10 mg Lithium/kg Ausgangssubstrat (bei Fermenter Typ 2 die doppelte Menge) am Ort der Substratzugabe zugesetzt. Daraufhin werden nach verschiedenen Zeiten (Zugabe erfolgt am Tag 1) an verschiedenen Stellen des Fermenters (am Anfang des Fermenters nach Substrateintritt sowie am Ende vor dem Restsubstrataustritt) Substratproben entnommen und diese mit Hilfe von induktiv gekoppelter Plasma-Atom-Emissionsspektroskopie nach den Methoden DIN EN ISO 13346 (S7a) und DIN EN ISO 11885 (E22) auf ihren Li-Gehalt hin analysiert. Nach einer vollständigen Durchmischung des Fermenterinhalts sollte der gemessene Li-Gehalt am vorderen Ende des Fermenters und am hinteren Ende des Fermenters gleich sein und einen Wert von ca. 10 mg/kg Ausgangssubstrat (ca. 20 mg/kg Ausgangssubstrat für Fermenter Typ 2) aufweisen. Die Ergebnisse von derartigen "Tracer-Tests" für 3 verschiedene Fermentertypen sind in Tabelle 1 dargestellt.

**Tabelle 1:**

| | Fermenter Typ 1 Lithium [mg/kg Ausgangssubstrat] | | Fermenter Typ 2 Lithium [mg/kg Ausgangssubstrat] | | Fermenter Typ 3 Lithium [mg/kg Ausgangssubstrat] | |
|---|---|---|---|---|---|---|
| Tag | vorne | hinten | vorne | hinten | vorne | hinten |
| 1 | 1,7 | 1,0 | 9,0 | 9,8 | 4,5 | 3,8 |
| 2 | 23,6 | 5,9 | 25,0 | 16,6 | | |
| 3 | 18,5 | 8,8 | 26,8 | 19,6 | 15,2 | 5,1 |
| 4 | 14,7 | 10,6 | 25,3 | 21,4 | | |
| 5 | | | 21,3 | 22,2 | 11,9 | 11,0 |
| 7 | | | | | 10,7 | 11,6 |
| 8 | 11,1 | 11,8 | 18,0 | 20,3 | 12,0 | 11,7 |
| 10 | | | | | 10,7 | 12,0 |
| 11 | | | 19,1 | 19,0 | | |

Es wurde beobachtet, dass eine vollständige Durchmischung des Fermenterinhalts und damit einhergehend eine gleichmäßige Verteilung des "Tracers" bei den Fermenter Typen 2 und 3 fünf Tage nach Zugabe des "Tracers" erreicht war, beim Fermenter Typ 1 erst nach 8 Tagen. Es ist davon auszugehen, dass der Zeitraum bis zu einer gleichmäßigen Verteilung von zugegebenen Mikroorganismen ebenfalls mehrere Tage beträgt.

### Langzeit-Fermentation zur Biogasproduktion mit oder ohne Zugabe von erfindungsgemäßen Mikroorganismen

Im Technikumsmaßstab konnte stabil mindestens eine Verdopplung der aus der Praxis bekannten durchschnittlichen Raumbelastung erreicht werden. Ein stabiler Betrieb wurde bei einer Raumbelastung von 8 kgoTS/m³d durch Zugabe von 2x10¹³ Zellen pro m³ und Woche erreicht. Dabei wurden an den in der Praxis üblichen Prozessparametern, wie Temperatur (40°C) und pH-Wert (6-9), bzw. Pufferkapazität nichts verändert.

Während des Fermentationsprozesses in verschiedenen Versuchsfermentern mit einem Volumen von 150 I bis 30000 I unter realistischen Anlagenbedingungen (T ~ 40 °C, pH 6 - 8, kontinuierliche Beschickung, ständige Durchmischung) wurde über einen Zeitraum von mehreren Monaten der zeitliche Verlauf der Raumbelastung des Fermenters in Kilogramm organischer Trockensubstanz je Kubikmeter je Tag (kgoTS/m³d) und der zeitliche Verlauf des produzierten Biogases bestimmt. Zur besseren Vergleichbarkeit der verschiedenen Versuchsanlagen, wurde für die Gasausbeute statt der spezifischen Biogasausbeute die Raumzeitausbeute (Normliter produziertes Biogas pro Liter Gärvolumen, [NI/I]) angegeben, bei der die Menge an erzeugtem Biogas auf das jeweilige Gärvolumen normiert wurde. Daneben wurde der zeitliche Verlauf der theoretisch erwarteten Gasproduktion berechnet.

Das Kuratorium für Technik und Bauwesen in der Landwirtschaft e. V. (KTBL) aber auch die Bundesforschungsanstalt für Landwirtschaft und die Fachagentur Nachwachsende Rohstoffe haben Richtwerte herausgegeben, die in etwa angeben, welche Mengen an Biogas in Abhängigkeit vom eingesetzten Substrat bei einer stabilen Fermentierung zu erwarten sind. Diese theoretischen Richtwerte können somit die Menge an theoretisch erzeugbarem Biogas widerspiegeln. Alternativ können auch von anderen Instituten in anderen Ländern herausgegebene Richtwerte verwendet werden. Für Maissilage, deren Anteile an oTS zwischen 22 % und 40 % lagen, wurden hierbei zu erwartende Gaserträge im Bereich von 533 NI/kgoTS und 650 NI/kgoTS genannt (in "Handreichung Biogasgewinnung und - nutzung", 2006, Hrsgb. Fachagentur Nachwachsende Rohstoffe e.V., Gülzow und KTBL Homepage, www.ktbl.de). Der zeitliche Verlauf der theoretischen Gasproduktion in [NI/d] wurde nach solchen Richtwerten berechnet, wobei in sämtlichen Versuchen hier bereits der sehr hohe Wert für einen theoretischen Gasertrag von 663 NI/kgoTS angenommen wurde. Die tatsächlich gemessenen Werte für die Anteile an oTS bei der eingesetzten Maissilage bewegten sich je nach Charge zwischen 30 und 40 % oTS.

Neben Gasausbeute und Raumbelastung wurden eine Reihe weiterer charakteristischer Parameter des Fermentationsprozesses wie Trockensubstanzgehalt des Fermenterinhalts, pH, Säurekonzentration (z.B. Essigsäure, Propionsäure, Buttersäure, Valeriansäure, Essigsäureäquivalent), Temperatur, spezifische Gasausbeute, Zusammensetzung des Biogases, Viskosität, Leitfähigkeit, Redoxpotential und die Konzentration an Nährstoffen und Spurenelementen gemessen.

### Langzeitfermentation in einem Fermenter vom Typ 4 unter Zusatz von Mikroorganismen Clostridium sporosphaeroides SBG3

Es handelt sich bei der Technikumsanlage um einen liegenden rechteckigen Pfropfenstromfermenter mit einem Volumen von 150 I. Der Fermenter wurde als 1-stufige Anlage betrieben.

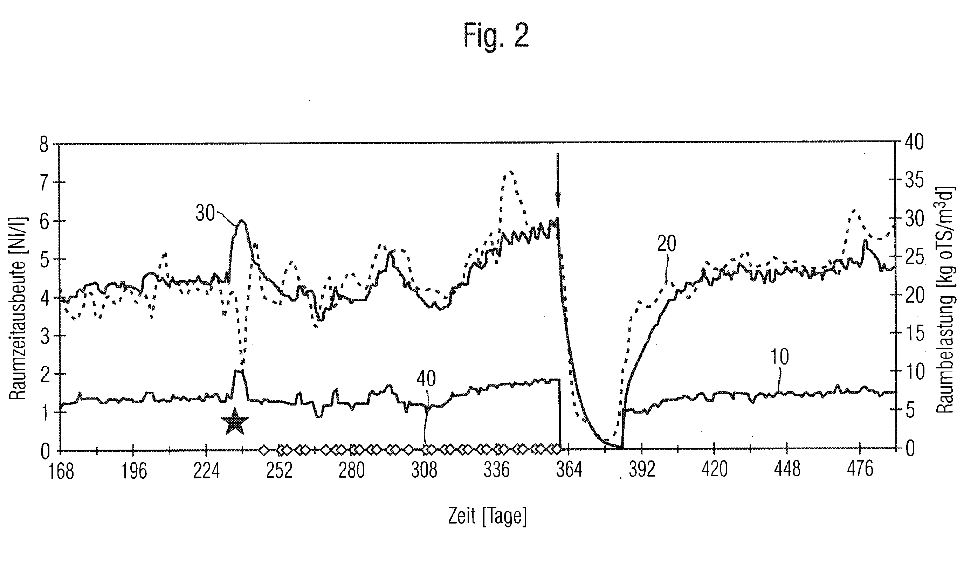

Die Figur 2 zeigt Messergebnisse verschiedener charakteristischer Parameter während eines Fermentationsprozesses in einem Versuchsfermenter mit und ohne Zugabe von Mikroorganismen des Stammes *Clostridium sporosphaeroides* SBG3. Die mit dem Bezugszeichen 10 versehene Kurve zeigt den zeitlichen Verlauf der Raumbelastung des Fermenters in Kilogramm organischer Trockensubstanz je Kubikmeter je Tag (kgoTS/m³d) und die mit dem Bezugszeichen 20 gekennzeichnete Kurve den zeitlichen Verlauf der gemessenen Raumzeitausbeute, gemittelt jeweils über 5 Tage (NI/I). Mit dem Bezugszeichen 30 ist der zeitliche Verlauf der theoretischen Gasproduktion in [NI/I] markiert.

Bis zum Betriebstag 246 wurde die Anlage ohne Zugabe von Mikroorganismen betrieben. Es konnte auf der Anlage in einer stabilen Fermentation eine recht hohe Raumbelastung zwischen 6 und 7 kgoTS/m³d erreicht werden, wobei die tatsächliche Gasausbeute jeweils etwas unterhalb der theoretischen blieb.

Ab dem Tag 232 (Sternsymbol) wurde versucht, die Raumbelastung bis hin zu 10 kgoTS/m³d steigern, was einen sofortigen Einbruch der Biogasproduktion zur Folge hatte. Nachdem die Raumbelastung wieder auf einen Wert von ca. 6 kgoTS/m³d zurückgefahren wurde, wurden ab Tag 246 zweimal pro Woche Kulturen von Mikroorganismen des Stammes *Clostridium sporosphaeroides* SBG3 zugegeben (zwischen 5 x 10¹⁰ und 2 x 10¹¹ Zellen pro Zugabe in einem Volumen von 30 bis 200 ml). Die Zeitpunkte der Zugabe von *Clostridium sporosphaeroides* SBG3 sind durch Rautensymbole 40 auf der X-Achse gekennzeichnet.

Anschließend stieg die tatsächliche Gasausbeute dauerhaft über den Wert der theoretisch berechneten an. Die Raumbelastung konnte in einem stabilen Fermentationsprozess stetig gesteigert und bis zum Tag 360 bis auf einen Wert von 9 kgoTS/m³d hochgefahren werden. Damit stieg auch die Raumzeitausbeute von anfänglichen 4 NI/I auf 6 NI/I an. Nach dem Tag 360 (Pfeil) wurde wegen einer Betriebspause die Substratzufuhr gestoppt und es wurden keine weiteren Mikroorganismen mehr zugegeben. Die Gasproduktion kam vollständig zum Erliegen.

Ab dem Tag 384 wurde die Anlage innerhalb eines Tages wieder auf eine Raumbelastung von 5 kgoTS/m³d hochgefahren ohne dass erneut Mikroorganismen zugegeben wurden. Bis etwa zum Tag 415 konnte die Raumbelastung auf einen Wert von 7 kgoTS/m³d gesteigert werden und blieb auch weiterhin bei konstant hohen Werten zwischen 7 und 8 kgoTS/m³d. Die Gasproduktion setzte daraufhin mit hoher Effizienz wieder ein und blieb über den gesamten Beobachtungszeitraum im Bereich oder über der theoretisch erwarteten Gasproduktion.

Dem Fachmann ist bekannt, dass ein derart problemloses Anfahren einer Anlage nach mehrwöchigem Stillstand sehr außergewöhlich ist. "Fishing"-Experimente nach dem erneuten Hochfahren der Anlage mit einer Oligonukleotidsonde gegen *Clostridium sporosphaeroides* SBG3 haben gezeigt, dass sich der Organismus im Fermenter etabliert hatte und mit einer Konzentration von etwa 0,5 % der Gesamtzahl an im Fermenterinhalt nachzuweisenden Bakterien vorhanden war. Dies erklärt auch die positiven Effekte der erhöhten Raumbelastung und Gasausbeute sowie der Stabilität des Fermentationsprozesses, die bereits vorher bei regelmäßiger Zugabe des Mikroorganismus *Clostridium sporosphaeroides* SBG3 zu beobachten waren.

### Langzeitfermentation in einem Fermenter vom Typ 1 unter Zusatz von Mikroorganismen Clostridium sporosphaeroides SBG3 sowie einer Mischung von Mikroorganismen der Stämme Clostridium sporosphaeroides SBG3, Clostridium sartagoformum SBG1a und Paenibacillus macerans SBG2

Es handelt sich bei der Technikumsanlage um einen liegenden rechteckigen Pfropfenstromfermenter mit einem Volumen von 150 I. Der Fermenter wurde als 2-stufige Anlage betrieben, da eine bautechnische Unterteilung des Fermenterraumes durch eine eingezogene Wand mit kleinflächigem Durchlass für den Substratstrom erfolgte. Dabei wurde der Fermenterraum quasi in 2 Reaktionsräume aufgeteilt, die im Volumenverhältnis 1:3 zueinander stehen, wobei der Raum direkt nach dem Substratzugabestutzen der kleinere ist und der Raum nach der Lochblende der größere. Messungen charakteristischer Fermentationsparameter ergaben, dass sich in den beiden Reaktionsräumen teilweise unterschiedliche Werte einstellten, so dass davon ausgegangen werden kann, dass auch verschiedene Substratumsetzungen bevorzugt in dem einen oder anderen Reaktorraum erfolgten.

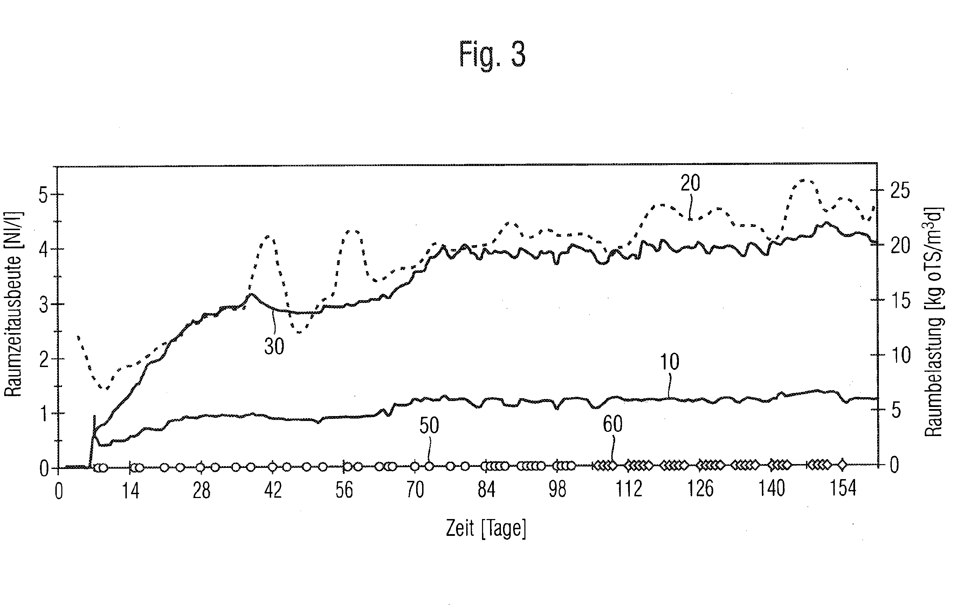

Die Figur 3 zeigt Messergebnisse verschiedener charakteristischer Parameter während eines Fermentationsprozesses in einem Versuchsfermenter vom Typ 1 mit Zugabe einer Mischung von Mikroorganismen bzw. von Mikroorganismen des Stammes *Clostridium sporosphaeroides* SBG3. Die mit dem Bezugszeichen 10 versehene Kurve zeigt den zeitlichen Verlauf der Raumbelastung des Fermenters in Kilogramm organischer Trockensubstanz je Kubikmeter je Tag (kg oTS/m³d) und die mit dem Bezugszeichen 20 gekennzeichnete Kurve den zeitlichen Verlauf der gemessenen Raumzeitausbeute, gemittelt jeweils über 5 Tage (NI/I). Mit dem Bezugszeichen 30 ist der zeitliche Verlauf der theoretischen Gasproduktion in [NI/I] markiert.

Die Anlage wurde komplett neu beschickt. Bereits nach einer Woche wurde eine Mischkultur aus in etwa gleichen Anteilen von Mikroorganismen der Stämme *Clostridium sporosphaeroides* SBG3, *Clostridium sartagoformum* SBG1a und *Paenibacillus macerans* SBG2 2 bis 5 mal pro Woche zugegeben. Die Zeitpunkte der Zugaben sind mit dem Bezugszeichen 50 gekennzeichnet. Es wurden jeweils ca. 3 g Zellpellet mit einer Zellzahl von etwa 10¹² Zellen zugegeben. In einem stabilen Fermentationsprozess konnte die Raumbelastung allmählich auf einen Wert von ca. 6 kgoTS/m³d gesteigert werden. Der erzielte Gasertrag entsprach in der Regel dem theoretisch erwarteten oder lag leicht darüber.

Ab dem Tag 106 wurde statt der Mischkultur nur noch eine Reinkultur des Stammes *Clostridium sporosphaeroides* SBG3 5 mal pro Woche zugesetzt (ebenfalls ca. 3 g Zellpellet mit einer Zellzahl von etwa 10¹² Zellen). Die Zeitpunkte der Zugaben sind mit dem Bezugszeichen 60 gekennzeichnet. Die Raumbelastung wurde bis zum Ende des Beobachtungszeitraumes auf einem hohen Niveau von 6 und 7 kgoTS/m³d konstant gehalten ohne dass die gemessenen Säurekonzentrationen in kritische Bereiche gelangten. Die gemessenen Anteile für die organische Trockensubstanz im Fermenterinhalt überschritten nie einen Wert von 12 % oTS, so dass auch bei hoher Raumbelastung eine gut rührbare nicht zu viskose Masse im Fermenter vorhanden war. In Kontrollexperimenten ohne Zusatz von Mikroorganismen konnte dieser Fermentertyp nie mit einer Raumbelastung von mehr als 5 kgoTS/m³d betrieben werden.

Bei Zugabe einer Reinkultur des Stammes *Clostridium sporosphaeroides* SBG3 erhöhte sich die tatsächlich gemessene Gasausbeute signifikant im Vergleich zu dem Zeitraum, in dem die Mischkultur zugegeben wurde. Für den Zeitraum, in dem 5 mal pro Woche die Mischkultur zugegeben wurde, wurde ein mittlerer Gasertrag von 4,1 NI/I gemessen, während nach Zugabe der Reinkultur von *Clostridium sporosphaeroides* SBG3 ein mittlerer Gasertrag von 4,6 NI/I bestimmt wurde, was einer Erhöhung um 12 % entspricht und wirtschaftlich durchaus von Bedeutung ist. Dieses Beispiel zeigt auch, dass bei Zugabe einer Reinkultur des Stammes *Clostridium sporosphaeroides* SBG3 die spezifische Biogasausbeute (Nm³/t) anstieg, da bei gleichbleibender Raumbelastung die Gasausbeute anstieg, das zugegebene Substrat also effizienter verwertet wurde.

### Langzeitfermentation in einem Fermenter vom Typ 3 unter Zusatz von Mikroorganismen Clostridium sporosphaeroides SBG3

Bei dieser Versuchsanlage handelt es sich um eine große Versuchsanlage im Technikumsmaßstab mit einem Volumen von 30 m³, die als liegender zylindrisch geformter Pfropfenstromfermenter aufgebaut ist. Sie wird als 1-stufige Anlage betrieben.

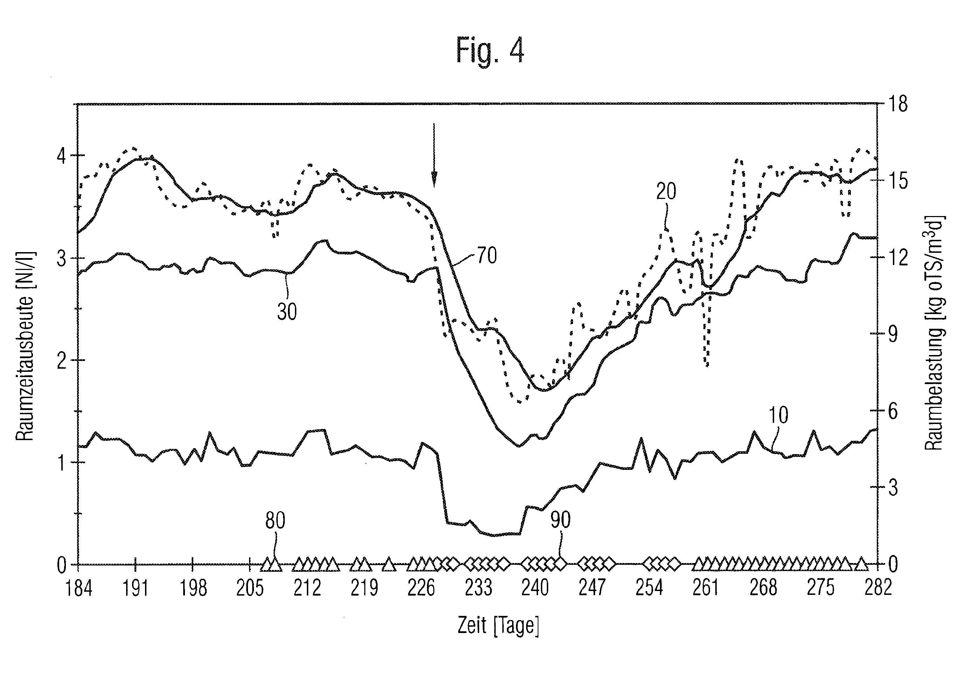

Die Figur 4 zeigt Messergebnisse verschiedener charakteristischer Parameter während eines Fermentationsprozesses in einem Versuchsfermenter vom Typ 3 mit Zugabe von Mikroorganismen des Stammes *Clostridium sartagoformum* SBG1a bzw. von Mikroorganismen des Stammes *Clostridium sporosphaeroides* SBG3. Die mit dem Bezugszeichen 10 versehene Kurve zeigt den zeitlichen Verlauf der Raumbelastung des Fermenters in Kilogramm organischer Trockensubstanz je Kubikmeter je Tag (kgoTS/m³d) und die mit dem Bezugszeichen 20 gekennzeichnete Kurve den zeitlichen Verlauf der gemessenen Raumzeitausbeute. Die Raumzeitausbeute gemittelt jeweils über 5 Tage (NI/I) ist mit dem Bezugszeichen 70 gekennzeichnet. Mit dem Bezugszeichen 30 ist der zeitliche Verlauf der theoretischen Gasproduktion in [NI/I] markiert.

Die Zeitpunkte der Zugaben von Mikroorganismen des Stammes *Clostridium sporosphaeroides* SBG3 sind mit dem Bezugszeichen 90 gekennzeichnet. Die Zeitpunkte der Zugaben von Mikroorganismen des Stammes *Clostridium sartagoformum* SBG1a sind mit dem Bezugszeichen 80 gekennzeichnet. Der Pfeil markiert den Beginn des "Absturzes" des Fermenters.

Die Biogasanlage wurde ab dem Betriebstag 184 mit einer Raumbelastung im Bereich von 4 bis 5 kgoTS/m³d betrieben. Wie in Figur 4 zu sehen ist, lag die erzielte Raumzeitausbeute an Biogas in diesem Versuch deutlich über der theoretisch erwarteten. Um den Prozess auf diesem hohen Niveau zu halten, wurde ab dem Tag 207 Reinkulturen von Mikroorganismen des Stammes *Clostridium sartagoformum* SBG1a zugegeben (4 bis 5 mal pro Woche jeweils ca. 100 g feuchtes Zellpellet aus einer 100 bis 200 I Anzucht, entspricht ca. 4 bis 8 mal 10¹³ Zellen). Der Prozess konnte so für mehr als 2 Wochen auf diesem hohen Niveau (Raumzeitausbeute im Bereich 3,5 bis 4 NI/I, ca. 15 % bis 30 % über dem theoretisch erwarteten Gasertrag) gehalten werden. Ab dem Tag 227 kam es jedoch zu einem plötzlichen Abfall der Gasbildung einhergehend mit einem rapiden Absinken der Raumbelastung auf einen Wert von nur noch etwas über 1 kgoTS/m³d, was einem "Absturz des Fermenters" gleichkommt. Dass der Fermentationsprozeß aus dem Gleichgewicht geriet, konnte man auch anhand weiterer Meßparameter wie pH-Wert (Absinken des pH-Wertes von ca. 7,7 bis 7,8 auf einen Wert von 6,0 am Tag 229) und den Messwerten für die sich akkumulierenden freien Fettsäuren (drastischer Anstieg der Werte für Essigsäure, Propionsäure, Buttersäure, Valeriansäure und Essigsäureäquivalent) beobachten. Um den Fermentationsprozeß wieder zu stabilisieren wurde vom Betriebstag 228 an jeweils eine Reinkultur von Mikroorganismen des Stammes *Clostridium sporosphaeroides* SBG3 zugegeben (4 bis 5 mal pro Woche, ca. 100 g feuchtes Zellpellet aus einer 100 bis 200 I Anzucht). Während des Absturzes, erreichte die erzielte Gasausbeute nur noch die theoretisch erwarteten (siehe Messreihen mit den Bezugszeichen 20 und 30), aber bereits ab Tag 230, also 2 Tage nach Zugabe der ersten Kultur von Mikroorganismen des Stammes *Clostridium sporosphaeroides* SBG3 liegen die gemessenen Gaserträge wieder über den theoretisch erwarteten. Eine Steigerung der Raumbelastung einhergehend mit kontinuierlich steigenden Gaserträgen war ab dem Tag 238 möglich. Die Zugabe von Mikroorganismen des Stammes *Clostridium sporosphaeroides* SBG3 wurde bis zum Tag 257 fortgeführt. Zu diesem Zeitpunkt war eine Raumbelastung von ca. 4 kgoTS/m³d erreicht, und die erzielte Gasausbeute lag stets deutlich über der theoretisch berechneten, aber das Effizienzniveau war immer noch etwas niedriger als vor dem Absturz. Die Zugabe von Mikroorganismen des Stammes *Clostridium sporosphaeroides* SBG3 wurde daraufhin gestoppt und es wurden ab Tag 260 erneut Mikroorganismen des Stammes *Clostridium sartagoformum* SBG1a zugegeben (4 bis 5 mal pro Woche jeweils ca. 100 g feuchtes Zellpellet aus einer 100 bis 200 I Anzucht). Es zeigte sich, dass dadurch die Raumausbeute noch mal leicht gesteigert werden konnte auf Werte zwischen 4,5 und 5 kgoTS/m³d und es ließ sich erneut ein Gasmehrertrag beobachten, der das Niveau vor dem Fermenterabsturz erreichte. Die erfindungsgemäße Zugabe von Mikroorganismen des Stammes *Clostridium sporosphaeroides* SBG3 erwies sich als geeignet, um einen Fermenter während eines Absturzes zu stabilisieren, so dass die Biogasproduktion wieder effizient weitergeführt werden konnte.

Durch die Zugabe der Reinkulturen von *Clostridium sporosphaeroides* SBG3 konnte bei Fermentationen die Raumbelastung bis auf einen Maximalwert von etwa 9 kgoTS/m³d gesteigert werden.

Parallel zur steigenden Raumbelastung konnte eine Steigerung des Biogasertrages beobachtet werden. Dabei war eine Übereinstimmung der erzeugten Biogasmenge in Normliter/Tag [NI/d] mit der theoretischen Gasproduktion in Normliter/Tag [NI/d] oder eine gegenüber der theoretischen Gasproduktion erhöhte Gasproduktion zu beobachten.

Die Raumbelastung der Anlage konnte infolge der Zugabe von *Clostridium sporosphaeroides* SBG3 weiter gesteigert werden. Dabei blieb der prozentuale Gehalt an Trockensubstanz beziehungsweise organischer Trockensubstanz nahezu konstant. Diese Beobachtung legt nahe, dass während der Fermentierung des Gärsubstrats keine Anhäufung von nicht-fermentierter organischer Trockensubstanz erfolgt. Die Zugabe von Reinkulturen von *Clostridium sporosphaeroides* SBG3 trägt also zu einer kontinuierlichen Umsetzung der enthaltenen Trockenmasse im Gärsubstrat bei, welche wiederum zu einer kontinuierlichen Fermentierung führt, indem die Anhäufung von Trockensubstanz vermindert wird.

In Phasen, in denen die Biogasanlage bei konstanter Raumbelastung betrieben wird, ist sogar eine Abnahme der Trockensubstanz zu beobachten, was darauf schließen lässt, dass die Mikroorganismen *Clostridium sporosphaeroides* SBG3 mit ihrer hydrolytischen Stoffwechselaktivität nicht nur die Anhäufung von Trockensubstanz im Fermenter vermindern, sondern auch die hydrolytische Umsetzung dieser Trockensubstanz verbessern.

In den beschriebenen Ausführungsbeispielen wurden Reinkulturen von *Clostridium sporosphaeroides* aber auch Mischkulturen mit einem Anteil an *Clostridium sporosphaeroides* verwendet. Insbesondere können Mischkulturen aus zwei oder drei Arten von Mikroorganismen ausgewählt aus der Gruppe bestehend aus *Paenibacillus macerans, Clostridium sartagoformum* und *Clostridium sporosphaeroides* zugegeben werden.

Die Zugabe des hydrolytisch aktiven, fermentativen Mikroorganismus *Clostridium sporosphaeroides* SBG3 zeigt einen positiven Effekt auf die Hydrolyse organischer Trockensubstanz. Durch die Zugabe von Mikroorganismen der Art *Clostridium sporosphaeroides* kann die Raumbelastung eines Fermenters unter ansonsten identischen Bedingungen von etwa 4 bis 5 kgoTS/m³d auf rund 6 bis 9 kgoTS/m³d gesteigert werden, ohne dass sich eine Instabilität des Fermentationsprozesses auch nur andeuten würde. Parallel zu der erhöhten Raumbelastung wird die Menge an gebildetem Biogas deutlich erhöht. Zudem steigt die spezifische Ausbeute an Biogas an, da deutlich mehr der organischen Trockensubstanz abgebaut wird als bei fehlender Zugabe von Mikroorganismen der Art *Clostridium sporosphaeroides.* Der Einsatz von Mikroorganismen der Art *Clostridium sporosphaeroides* führt zu einer dramatischen Verbesserung von Effizienz und Wirkungsgrad von Biogasanlagen.

## Patentansprüche

1. Verfahren zur Erzeugung von Biogas aus Biomasse, **dadurch gekennzeichnet, dass** der Biomasse ein Mikroorganismus der Art *Clostridium sporosphaeroides* in Form einer Kultur von Mikroorganismen zugesetzt wird, wobei der Mikroorganismus der Art *Clostridium sporosphaeroides* zumindest 1 % der Gesamtzahl an in der Kultur vorhandenen Mikroorganismen ausmacht.

2. Verfahren nach Anspruch 1, **dadurch gekennzeichnet, dass** in der Kultur von Mikroorganismen der Mikroorganismus der Art *Clostridium sporosphaeroides* zumindest 10 % der Gesamtzahl an in der Kultur vorhandenen Mikroorganismen ausmacht.

3. Verfahren nach einem der vorhergehenden Ansprüche, **dadurch gekennzeichnet, dass** eine Reinkultur des Mikroorganismus der Art *Clostridium sporosphaeroides* zugesetzt wird.

4. Verfahren nach einem der vorhergehenden Ansprüche, **dadurch gekennzeichnet, dass** der Mikroorganismus der Art *Clostridium sporosphaeroides* der Biomasse als Bestandteil zumindest einer immobilisierten Kultur von Mikroorganismen zugesetzt wird.

5. Verfahren nach einem der vorhergehenden Ansprüche, **dadurch gekennzeichnet, dass** zeitnah zur Zugabe des Mikroorganismus der Art *Clostridium sporosphaeroides* zusätzliche Biomasse in den Fermentierungsreaktor gegeben wird, wobei die Raumbelastung im Fermentierungsreaktor durch kontinuierliche Zugabe von Biomasse kontinuierlich gesteigert wird.

6. Verfahren nach einem der vorhergehenden Ansprüche, **dadurch gekennzeichnet, dass** die Erzeugung von Biogas aus Biomasse bei einer Raumbelastung von ≥ 0,5 kgoTS/m³d, bevorzugt ≥ 4,0 kgoTS/m³d, besonders bevorzugt ≥ 8,0 kgoTS/m³d, durchgeführt wird.

7. Verfahren nach einem der vorhergehenden Ansprüche, **dadurch gekennzeichnet, dass** der Mikroorganismus der Art *Clostridium sporosphaeroides* in einer Menge zu dem Gärsubstrat zugegeben wird, dass nach Zugabe der Anteil des Mikroorganismus der Art *Clostridium sporosphaeroides* zwischen 10⁻⁴ % und 10 % der Gesamtzahl an in dem Gärsubstrat anwesenden Mikroorganismen ausmacht.

8. Verfahren nach Anspruch 7, **dadurch gekennzeichnet, dass** der Mikroorganismus der Art *Clostridium sporosphaeroides* in einer Menge zu dem Gärsubstrat zugegeben wird, dass nach Zugabe der Anteil des Mikroorganismus der Art *Clostridium sporosphaeroides* zwischen 10⁻³ % und 1 % der Gesamtzahl an in dem Gärsubstrat anwesenden Mikroorganismen ausmacht.

9. Verfahren nach einem der vorhergehenden Ansprüche, **dadurch gekennzeichnet, dass** zusätzlich Mikroorganismen der Art *Clostridium sartagoformum* zugegeben werden.

10. Verfahren nach Anspruch 9, **dadurch gekennzeichnet, dass** es sich um Mikroorganismen des Stammes *Clostridium sartagoformum* SBG1a (hinterlegungsnummer DSM 22578) handelt.

11. Verfahren nach einem der vorhergehenden Ansprüche, **dadurch gekennzeichnet, dass** zusätzlich Mikroorganismen der Art *Paenibacillus macerans* zugegeben werden.

12. Verfahren nach Anspruch 11, **dadurch gekennzeichnet, dass** es sich um Mikroorganismen des Stammes *Paenibacillus macerans* SBG2 (hinterlegungsnummer DSM 22569) handelt.

13. Stamm des Mikroorganismus *Clostridium sporosphaeroides* SBG3, der bei der DSMZ unter der Nr. DSM 22577 hinterlegt ist.

14. Mikroorganismus mit einer Nukleinsäure, die eine Nukleotidsequenz aufweist, **dadurch gekennzeichnet, dass** die Nukleotidsequenz einen Sequenzbereich enthält, der mehr als 98,5 % Sequenzidentität mit der Nukleotidsequenz SEQ ID Nr. 1 aufweist.

15. Kultur von Mikroorganismen geeignet zum Einsatz in einem Verfahren zur Erzeugung von Biogas aus Biomasse, **dadurch gekennzeichnet, dass** in der Kultur von Mikroorganismen ein Mikroorganismus *Clostridium sporosphaeroides* gemäß Anspruch 13 oder 14 anwesend ist, wobei der Mikroorganismus *Clostridium sporosphaeroides* zumindest 1 % der Gesamtzahl an in der Kultur vorhandenen Mikroorganismen ausmacht.

16. Kultur von Mikroorganismen nach Anspruch 15, **dadurch gekennzeichnet, dass** der Mikroorganismus *Clostridium sporosphaeroides* zumindest 10 % der Gesamtzahl an in der Kultur vorhandenen Mikroorganismen ausmacht.

17. Kultur von Mikroorganismen nach Anspruch 16, **dadurch gekennzeichnet, dass** es sich um eine Reinkultur des Mikroorganismus *Clostridium sporosphaeroides* handelt.

18. Kultur von Mikroorganismen nach zumindest einem der Ansprüche 15 bis 17, **dadurch gekennzeichnet, dass** es sich um eine immobilisierte Kultur von Mikroorganismen handelt.

## Claims

1. A process for the generation of biogas from biomass, **characterized in that** a microorganism of the species *Clostridium sporosphaeroides* is added to the biomass in the form of a culture of microorganisms, wherein the microorganism of the species *Clostridium sporosphaeroides* makes up at least 1% of the total number of microorganisms present in the culture.

2. The process as claimed in claim 1, **characterized in that**, in the culture of microorganisms, the microorganism of the species *Clostridium sporosphaeroides* makes up at least 10% of the total number of microorganisms present in the culture.

3. The process as claimed in one of the previous claims, **characterized in that** a pure culture of the microorganism of the species *Clostridium sporosphaeroides* is added.

4. The process as claimed in one of the previous claims, **characterized in that** the microorganism of the species *Clostridium sporosphaeroides* is added to the biomass as a component of at least one immobilized culture of microorganisms.

5. The process as claimed in one of the previous claims, **characterized in that**, close to the time of the addition of the microorganism of the species *Clostridium sporosphaeroides,* additional biomass is added to the fermentation reactor, whereby the volume loading in the fermentation reactor is continuously increased by continuous addition of biomass.

6. The process as claimed in one of the previous claims, **characterized in that** the generation of biogas from biomass is performed at a volume loading of ≥ 0.5 kg oDS/m³d, preferably ≥ 4.0 kg oDS/m³d, particularly preferably ≥ 8.0 kg oDS/m³d.

7. The process as claimed in one of the previous claims, **characterized in that** the microorganism of the species *Clostridium sporosphaeroides* is added to the fermentation substrate in a quantity such that, after addition, the content of the microorganism of the species *Clostridium sporosphaeroides* makes up between 10⁻⁴% and 10% of the total number of microorganisms present in the fermentation substrate.

8. The process as claimed in claim 7, **characterized in that** the microorganism of the species *Clostridium sporosphaeroides* is added to the fermentation substrate in a quantity such that, after addition, the content of the microorganism of the species *Clostridium sporosphaeroides* makes up between 10⁻³% and 1% of the total number of microorganisms present in the fermentation substrate.

9. The process as claimed in one of the previous claims, **characterized in that**, microorganisms of the species *Clostridium sartagoformum* are added additionally.

10. The process as claimed in claim 9, **characterized in that**, the microorganisms originate of the strain *Clostridium sartagoformum* SBG1 (deposit number DSM 22578).

11. The process as claimed in one of the previous claims, **characterized in that**, microorganisms of the species *Paenibacillus macerans* are added additionally.

12. The process as claimed in claim 11, **characterized in that**, the microorganisms originate of the strain *Paenibacillus macerans* SBG2 (deposit number DSM 22569) .

13. Strain of the microorganism *Clostridium sporosphaeroides SBG1* which is deposited at the DSMZ under the No. DSM 22577.

14. A microorganism with a nucleic acid, which has a nucleotide sequence, **characterized in that** the nucleotide sequence contains a sequence region which has more than 98.5% sequence identity with the nucleotide sequence SEQ ID No. 1.

15. A culture of microorganisms suitable for use in a process for the generation of biogas from biomass, **characterized in that**, in the culture of microorganisms, a microorganism *Clostridium sporosphaeroides* as claimed in claims 13 or 14 is present, wherein the microorganism *Clostridium sporosphaeroides* makes up at least 1% of the total number of microorganisms present in the culture.

16. The culture of microorganisms as claimed in claim 15, **characterized in that** the microorganism *Clostridium sporosphaeroides* makes up at least 10% of the total number of microorganisms present in the culture.

17. The culture of microorganisms as claimed in claim 16, **characterized in that** it is a pure culture of the microorganism *Clostridium sporosphaeroides.*

18. The culture of microorganisms as claimed in at least one of claims 15 to 17, **characterized in that** it is an immobilized culture of microorganisms.

## Revendications

1. Procédé de production de biogaz à partir de biomasse, **caractérisé en ce qu'**un microorganisme de l'espèce *Clostridium sporosphaeroides* est ajouté à la biomasse sous la forme d'une culture de microorganismes, le microorganisme de l'espèce *Clostridium sporosphaeroides* constituant au moins 1% du nombre total des microorganismes présents dans la culture.

2. Procédé selon la revendication 1, **caractérisé en ce que** le microorganisme de l'espèce *Clostridium sporosphaeroides* constitue au moins 10% du nombre total des microorganismes présents dans la culture de microorganismes.

3. Procédé selon l'une des revendications précédentes, **caractérisé en ce qu'**un bouillon de culture du microorganisme de l'espèce *Clostridium sporosphaeroides* est utilisé.

4. Procédé selon l'une des revendications précédentes, **caractérisé en ce que** le microorganisme de l'espèce *Clostridium sporosphaeroides* est ajouté à la biomasse en tant que constituant d'au moins une culture immobilisée de microorganismes.

5. Procédé selon l'une des revendications précédentes, **caractérisé en ce qu'**au moment de l'ajout du microorganisme de l'espèce *Clostridium sporosphaeroides,* de la biomasse supplémentaire est ajoutée dans le réacteur de fermentation, l'occupation d'espace dans le réacteur de fermentation étant continuellement augmentée par l'ajout continuel de biomasse.

6. Procédé selon l'une des revendications précédentes, **caractérisé en ce que** la production de biogaz à partir de la biomasse est effectuée avec une occupation d'espace supérieure ou égale à 0,5 kg d'oTS/m3d, de préférence supérieure ou égale à 4,0 kg d'oTS/m3d, de façon particulièrement préférée supérieure ou égale à 8,0 kg d'oTS/m3d.

7. Procédé selon l'une des revendications précédentes, **caractérisé en ce que** le microorganisme de l'espèce *Clostridium sporosphaeroides* est ajouté au substrat de fermentation en une quantité telle qu'après ajout de la proportion de microorganisme de l'espèce *Clostridium sporosphaeroides,* il représente entre 10-4% et 10% du nombre total des microorganismes présents dans le substrat de fermentation.

8. Procédé selon la revendication 7, **caractérisé en ce que** le microorganisme de l'espèce *Clostridium sporosphaeroides* est ajouté au substrat de fermentation en une quantité telle qu'après ajout de la proportion de microorganisme de l'espèce *Clostridium sporosphaeroides,* il représente entre 10-3% et 1% du nombre total des microorganismes présents dans le substrat de fermentation.

9. Procédé selon l'une des revendications précédentes, **caractérisé en ce qu'**en outre des microorganismes de l'espèce *Clostridium sartagoformum* sont ajoutés.

10. Procédé selon la revendication 9, **caractérisé en ce qu'**il s'agit des microorganismes de la souche *Clostridium sartagoformum* SBG1 (numéro du dépôt DSM 22578).

11. Procédé selon l'une des revendications précédentes, **caractérisé en ce qu'**en outre des microorganismes de l'espèce *Paenibacillus macerans* sont ajoutés.

12. Procédé selon la revendication 11, **caractérisé en ce qu'**il s'agit des microorganismes de la souche *Paenibacillus macerans* SBG2 (numéro du dépôt DSM 22569).

13. Souche du microorganisme *Clostridium sporosphaeroides* SBG1, déposée auprès de la DSMZ sous le numéro DSM 22577.

14. Microorganisme ayant un acide nucléique présentant une séquence de nucléotides, **caractérisé en ce que** la séquence de nucléotides contient un domaine de séquence qui présente plus de 98,5% d'identité de séquence avec la séquence de nucléotides SEQ ID n°1.

15. Culture de microorganismes appropriée pour une utilisation dans un procédé de production de biogaz à partir de biomasse, **caractérisée en ce qu'**un microorganisme *Clostridium sporosphaeroides* selon la revendication 13 ou 14 est présent dans la culture de microorganismes, le microorganisme *Clostridium sporosphaeroides* constituant au moins 1 % du nombre total des microorganismes présents dans la culture.

16. Culture de microorganismes selon la revendication 15, **caractérisée en ce que** le microorganisme *Clostridium sporosphaeroides* constitue au moins 10% du nombre total des microorganismes présents dans la culture.

17. Culture de microorganismes selon la revendication 16, **caractérisée en ce qu'**il s'agit d'un bouillon de culture du microorganisme *Clostridium sporosphaeroides.*

18. Culture de microorganismes selon l'une au moins des revendications 15 à 17, **caractérisée en ce qu'**il s'agit d'une culture immobilisée de microorganismes.


### SEQUENCE LISTING

<110> Schmack Biogas AG
<120> clostridium sporosphaeroides zur Erzeugung von Biogas
<130> SCM068WO
<150> PCT/DE2008/075017
   <151> 2008-12-23
<150> DE 10 2009 003 587.7
   <151> 2009-03-07
<160> 1
<170> PatentIn version 3.3
<210> 1
   <211> 1409
   <212> DNA
   <213> Clostridium sporosphaeroides SBG3
<400> 1
   cagatcacct tagacacgtc ccccttgcgg ttagactatt ggcttcgggt attaccggct     60
   cccatggtgt gacgggcggt gtgtaccagg cccgggaacg tattcaccgc ggcatgatga    120
   tccgcgatta ctagcaattc caacttcatg taggcgggtt gcagcctaca atccgaactg    180
   agatcgcttt tgggggtttg ctccacctcg cggcttggct tccctctgtt agcgaccatt    240
   gtagtacgtg tgtagcccag gtcataaggg gcatgatgat ttgacgtcgt ccccaccttc    300
   ctccgttttg tcaacggcag tctgattaga gtgctcttgc gtacgactaa tcacaagggt    360
   tgcgctcgtt gcgggactta acccaacatc tcacgacacg agctgacgac aaccatgcac    420
   cacctgtctc aactttcccc gaagccacct aatgcatctc tgcctcgtta gttggatgtc    480
   aagacctggt aaggttcttc gcgttgcttc gaattaaacc acatactcca ctgcttgtgc    540
   gggcccccgt caattccttt gagtttcaac ctggcggccg tactccccag gtggatcact    600
   tattgtgtta actccggcac ggaaggggtc agacccccca cacctagtaa tcatcgttta    660
   cggcatggac taccagcgta tctaatcctg tttgctaccc aggcttccgt gcctcagcgt    720
   cagttaaagc ccagtaggcc gccttcgcca ctggtgttcc tcccgatctc tacgcatttc    780
   aacgctacac cgggaattcc gcctacatct acgtcaatca agcctcccag tttcgaacgc    840
   aatttgtggg ttaagcccac agctctcacg cccgacttaa aaagccgcta cgcacccttt    900
   acacccagta aatccggaca acgcttgctc cctacgtatt accgcggctg ctggcacgta    960
   gttagccgga gcttcctcct tggctaccgt cattatcttc accaaggaca gaggtttaca   1020
   atccgaagac cgtcttccct cacgcggcat tgctgcatca gagtttcctc cattgtgcaa   1080
   tatcccccac tgctgcctcc cgtaggagtc tgggccgtgt ctcagtccca atgtggccgt   1140
   tcagtctctc aacccggcta ccgatcgtcg ctttggtggg cctttacccc gccaactggc   1200
   taatcggacg cgagtccatc tttcagcgga ttgctccttt gataccaagg ccatgcgacc   1260
   ccaatatatc atgcggtatt agcgttcttt tcagaacgtt atccccctct gaaaggcagg   1320
   ttactcacgc gttactcacc cgtccgccac taagtaagag ctaagcaagc ttctctcttc   1380
   tccgtcgact tgcattttga acgtcgcga                                     1409